# EUROPEAN PATENT SPECIFICATION

(11) **EP 4 125 441 B1**
(45) Date of publication and mention of the grant of the patent: **25.02.2026**
(21) Application number: 21723041.6
(22) Date of filing: 05.04.2021
(51) Int. Cl.: C12R 1/01, A23L 33/135, A61K 35/741, A61P 3/04, A61P 3/08, A61P 3/10, C12N 1/20

(54) **COMPOSITION COMPRISING BACTERIAL STRAINS FOR IMPROVING METABOLIC HEALTH**
ZUSAMMENSETZUNG MIT AKKERMANSIA DSM 33459 ZUR VERBESSERUNG DER STOFFWECHSELGESUNDHEIT
COMPOSITION COMPRENANT AKKERMANSIA DSM 33459 POUR AMÉLIORER LA SANTÉ MÉTABOLIQUE

(30) Priority: 03.04.2020 US 202063004617 P
(43) Date of publication of application: 08.02.2023
(60) Divisional application: 25160100.1
(73) Proprietor: International N&H Denmark ApS, 2800 Kongens Lyngby (DK)
(72) Inventor: FORSSTEN, Sofia, 02460 Kantvik (FI); HASSELWANDER, Oliver, Marlborough Wiltshire SN8 1AA (GB); HIBBERD, Ashley, Ann, Saint Louis, MO 63139 (US); JENSEN, Henrik, Max, 8220 Brabrand (DK); KANE, Helene, M A, Woodbury, NJ 08096 (US); KIM, Hye-Sook, Hockessin, DE 19707 (US); KUMAR, Ritesh, Wilmington, DE 19803 (US); OUWEHAND, Arthur, 10210 Inga (FI); RASINKANGAS, Pia, Tuulikki, 00320 Helsinki (FI); ROUVIERE, Pierre, E, Wilmington, DE 19803 (US); WANG, Qiong, Palo Alto, CA 94304 (US); STAHL, Buffy, L, Madison, WI 53716 (US)
(74) Representative: D Young & Co LLP
(86) International application number: PCT/US2021/025730
(87) International publication number: WO 2021/203083

(56) References cited:
- EP-A1- 3 165 227
- EP-A1- 3 165 227
- WO-A1-2012/142605
- WO-A1-2014/196913
- WO-A1-2016/185469
- WO-A1-2017/042347
- WO-A1-2017/042347
- WO-A1-2019/028402
- WO-A1-2019/051380
- CA-A1- 3 114 471
- CA-A1- 3 114 471
- KIRMIZ NINA ET AL: "Comparative Genomics Guides Elucidation of Vitamin B 12 Biosynthesis in Novel Human-Associated Akkermansia Strains", APPLIED AND ENVIRONMENTAL MICROBIOLOGY, vol. 86, no. 3, February 2020 (2020-02-01), US, pages 1 - 16, XP055936628, ISSN: 0099-2240, DOI: 10.1128/AEM.02117-19
- ASHOK TEJASWINI ET AL: "Role of Vitamin B12 and Folate in Metabolic Syndrome", CUREUS, 6 October 2021 (2021-10-06), US, XP093119787, ISSN: 2168-8184, DOI: 10.7759/cureus.18521
- EDGAR ROBERT C: "Updating the 97% identity threshold for 16S ribosomal RNA OTUs", BIOINFORMATICS, vol. 34, no. 14, 28 February 2018 (2018-02-28), GB, pages 2371 - 2375, XP093119907, ISSN: 1367-4803, DOI: 10.1093/bioinformatics/bty113
- KIRMIZ NINA ET AL: "Comparative Genomics Guides Elucidation of Vitamin B 12 Biosynthesis in Novel Human-Associated Akkermansia Strains", APPLIED AND ENVIRONMENTAL MICROBIOLOGY, vol. 86, no. 3, February 2020 (2020-02-01), US, pages 1 - 16, XP055936628, ISSN: 0099-2240, DOI: 10.1128/AEM.02117-19
- ASHOK TEJASWINI ET AL: "Role of Vitamin B12 and Folate in Metabolic Syndrome", CUREUS, 6 October 2021 (2021-10-06), US, XP93119787, ISSN: 2168-8184, DOI: 10.7759/cureus.18521
- EDGAR ROBERT C: "Updating the 97% identity threshold for 16S ribosomal RNA OTUs", BIOINFORMATICS, vol. 34, no. 14, 28 February 2018 (2018-02-28), GB, pages 2371 - 2375, XP93119907, ISSN: 1367-4803, DOI: 10.1093/bioinformatics/bty113
- SAMOCHA-BONET DORIT ET AL: "Prevention and Treatment of Type 2 Diabetes: A Pathophysiological-Based Approach", TRENDS IN ENDOCRINOLOGY AND METABOLISM, ELSEVIER SIENCE PUBLISHING , NEW YORK , NY, US, vol. 29, no. 6, 14 April 2018 (2018-04-14), pages 370 - 379, XP085397182, ISSN: 1043-2760, DOI: 10.1016/J.TEM.2018.03.014
- LEE JENNIFER H. ET AL: "Circulating Resistin Levels Are Not Associated with Obesity or Insulin Resistance in Humans and Are Not Regulated by Fasting or Leptin Administration: Cross-Sectional and Interventional Studies in Normal, Insulin-Resistant, and Diabetic Subjects", JOURNAL OF CLINICAL ENDOCRINOLOGY AND METABOLISM, vol. 88, no. 10, October 2003 (2003-10-01), US, pages 4848 - 4856, XP055853482, ISSN: 0021-972X, DOI: 10.1210/jc.2003-030519

## Description

### CROSS-REFERENCE TO RELATED APPLICATIONS

This application claims priority to U.S. Provisional Patent Application No. 63/004,617, filed April 3, 2020.

### FIELD OF THE INVENTION

Provided herein, *inter alia,* are bacterial compositions useful for improving metabolic health in subjects as well as methods for making and using the same.

### BACKGROUND

The human gastrointestinal tract contains a complex and diverse ecosystem of microorganisms. Intestinal bacteria are not only commensal, but they also undergo a symbiotic co-evolution with their host. The interaction between gut microbiota and host is complex. Beneficial intestinal bacteria have numerous important functions and they directly or indirectly affect various physiological functions in the host, *e.g.*, they provide nutrients to their host, prevent infections caused by intestinal pathogens, and modulate a normal immunological response. It is established that imbalance in the microbiota composition results in various disease states in the host. Therefore, modification of the intestinal microbiota in order to achieve, restore, and maintain favorable balance in the ecosystem, as well as the activity of microorganisms present in the gastrointestinal tract, is necessary for maintaining and improving the health condition of the host.

First-generation probiotics are live microorganisms mainly derived from the genera *Lactobacillus* and *Bifidobacterium,* which are often minor constituents of the digestive tract or originate from use as dairy starter cultures. Traditionally, first-generation probiotics are mainly targeted at gut and immune health. Some, such as *B. lactis* B420, have been shown exert beneficial activity also in relation to metabolic health, *e.g.,* in reduction in body fat mass and some improvement for blood glucose and insulin. However, current first-generation probiotics do not seem to provide an optimal solution with respect to glucose and insulin metabolism, *i.e.,* as potential treatments or preventative agents for type 2 diabetes, pre-diabetes, or metabolic syndrome.

What is needed, therefore, are additional microorganisms identified based on their natural occurrence in the digestive tract of metabolically healthy individuals and which have been selected based on their ability to maintain and optimize metabolic health and prevent disease.

The subject matter disclosed herein addresses these needs and provides additional benefits as well.

CA3114471A1 discloses an *Akkermansia muciniphila* strain and use thereof in preventing and treating metabolic diseases. This strain is named *Akkermansia muciniphila* SSYD-3 and the accession number thereof is CGMCC No. 14764.

EP3165227A1 discloses a pharmaceutical and food composition for treating, preventing, or alleviating a metabolic disease, containing, as an active ingredient, extracellular vesicles derived from *Akkermansia muciniphila.* The composition disclosed can be used as a pharmaceutical/food composition and the like for treating, preventing, or alleviating metabolic diseases, in particular metabolic diseases such as obesity, diabetes, hyperlipidemia, arteriosclerosis, and hypertension, occurring or exacerbated due to a high fat diet.

### SUMMARY

The invention is set out in the appended set of claims. Accordingly, all aspects and embodiments of the invention are as set out in the claims. Only compositions comprising *Akkermansia* sp DSM 33459 or EVs derived from *Akkermansia* sp. DSM 33459, kits, methods for making the composition and the composition for use, as set out in the appended set of claims, are within the scope of the present invention. Any disclosure set forth herein relating to a composition or a method of preparing a composition comprising *Eubacterium eligens, Intestimonas massiliensis* or *Prevotella copri,* or EVs derived therefrom, wherein said composition does not also comprise *Akkermansia* sp DSM 33459, or EVs derived therefrom, are not of the invention but are considered as useful for understanding the invention.

Any references to methods of treatment by therapy or methods of administering a composition disclosed herein to a subject, in the description and examples of this description, are to be interpreted as references to compounds, pharmaceutical compositions and medicaments of the present invention for use in those methods. Methods of treatment are excluded from patentability under Art. 53(c) EPC and are not within the scope of protection.

Provided herein, *inter alia,* are compositions comprising one or more biologically pure strains of bacteria according to the claims and methods of making and using the same to treat and/or prevent one or more obesity related disorders selected from: obesity, metabolic syndrome, diabetes mellitus, insulin deficiency-related disorders, insulin-resistance related disorders, glucose intolerance, abnormal lipid metabolism, non-alcoholic fatty liver disease, hepatic steatosis, leptin resistance, reduced resistin levels, and/or cardiovascular disease in a subject in need thereof.

Described herein is a composition comprising at least one or more of (a) a biologically pure strain of *Eubacterium eligens;* (b) a biologically pure strain of *Intestinimonas massiliensis;* (c) a bacterial strain having a 16S ribosomal RNA sequence displaying at least 97.0% sequence similarity to a 16S ribosomal RNA sequence of *Prevotella copri* deposited at the German Collection of Microorganisms and Cell Cultures (DSM) under number DSM 33457; and/or (d) a biologically pure strain of an *Akkermansia sp.,* wherein said *Akkermansia sp.* is not (i) *Akkermansia muciniphila;* or (ii) *Akkermansia glycaniphilia.* Described herein, genome-wide average nucleotide identity (gANI) between said *Akkermansia sp.* and (i) *Akkermansia muciniphila;* or (ii) *Akkermansia glycaniphilia* is less than about 95%. As described herein, the composition comprises (a) a bacterial strain having a 16S ribosomal RNA sequence displaying at least 97.0% sequence similarity to a 16S ribosomal RNA sequence of *E. eligens* deposited at DSM under number DSM 33458; and/or (b) a bacterial strain having a 16S ribosomal RNA sequence displaying at least 97.0% sequence similarity to a 16S ribosomal RNA sequence of *I*. *massiliensis* deposited at DSM under number DSM 33460. As described herein, the composition comprises (a) the *E. eligens* strain deposited at DSM under number DSM 33458 or a live strain having all of the identifying characteristics of the *E. eligens* strain deposited at DSM under number DSM 33458; (b) the *I. massiliensis* strain deposited at DSM under number DSM 33460 or a live strain having all of the identifying characteristics of the *I*. *massiliensis* strain deposited at DSM under number DSM 33460; (c) the *P. copri* strain deposited at DSM under number DSM 33457 or a live strain having all of the identifying characteristics of the *P. copri* strain deposited at DSM under number DSM 33457; and/or (d) the *Akkermansia sp.* deposited at DSM under number DSM 33459 or a live strain having all of the identifying characteristics of the *Akkermansia sp.* deposited at DSM under number DSM 33459 either (A) alone; and/or (B) in combination with a culture supernatant derived from one or more of these strains. As described herein, the composition comprises (b) a biologically pure strain of *Intestinimonas massiliensis;* and (d) a biologically pure strain of an *Akkermansia sp.,* wherein said *Akkermansia sp.* is not (i) *Akkermansia muciniphila*; or (ii) *Akkermansia glycaniphilia.* As described herein, the composition comprises (b) a bacterial strain having a 16S ribosomal RNA sequence displaying at least 97.0% sequence similarity to a 16S ribosomal RNA sequence of *I. massiliensis* deposited at DSM under number DSM 33460. Described herein, genome-wide average nucleotide identity (gANI) between said *Akkermansia sp.* and *(i) Akkermansia muciniphila;* or (ii) *Akkermansia glycaniphilia* is less than about 95%. In some embodiments of any of the embodiments disclosed herein, the composition comprises (b) the *I. massiliensis* strain deposited at DSM under number DSM 33460 or a live strain having all of the identifying characteristics of the *I*. *massiliensis* strain deposited at DSM under number DSM 33460; and (d) the *Akkermansia sp.* deposited at DSM under number DSM 33459. Only compositions comprising *Akkermansia sp* DSM 33459 as set forth in the claims are within the scope of the present invention. In some embodiments of any of the embodiments disclosed herein, the composition is formulated for oral administration. In some embodiments of any of the embodiments disclosed herein, the composition is lyophilized or freeze dried. In some embodiments of any of the embodiments disclosed herein, the composition is encapsulated or coated. In some embodiments of any of the embodiments disclosed herein, the composition is a food product, food ingredient, dietary supplement, or medicament. In some embodiments disclosed herein, at least about 1 x 10⁴ CFU/g composition to at least about 1 x 10¹² CFU/g composition of bacteria is present in the composition. In some embodiments disclosed herein, the composition is a probiotic. In some embodiments of any of the embodiments disclosed herein, the composition has been pasteurized or heat treated. In some embodiments of any of the embodiments disclosed herein, the composition is a pharmaceutical composition and further comprises at least one pharmaceutically acceptable carrier and/or excipient.

Described herein is a composition comprising isolated bacterial extracellular vesicles (EVs) derived from at least one or more of (a) a biologically pure strain of *Eubacterium eligens*; (b) a biologically pure strain of *Intestinimonas massiliensis*; (c) a bacterial strain having a 16S ribosomal RNA sequence displaying at least 97.0% sequence similarity to a 16S ribosomal RNA sequence of *Prevotella copri* deposited at the German Collection of Microorganisms and Cell Cultures (DSM) under number DSM 33457; and/or (d) a biologically pure strain of an *Akkermansia sp.,* wherein said *Akkermansia sp.* is not *(i) Akkermansia muciniphila;* or (ii) *Akkermansia glycaniphilia.* As described herein, the composition further comprises one or more bacteria from (a), (b), (c), and/or (d). Described herein, genome-wide average nucleotide identity (gANI) between said *Akkermansia sp.* and (i) *Akkermansia muciniphila;* or (ii) *Akkermansia glycaniphilia* is less than about 95%. As described herein, the composition comprises (a) EVs derived from a bacterial strain having a 16S ribosomal RNA sequence displaying at least 97.0% sequence similarity to a 16S ribosomal RNA sequence of *E. eligens* deposited at DSM under number DSM 33458; and/or (b) EVs derived from a bacterial strain having a 16S ribosomal RNA sequence displaying at least 97.0% sequence similarity to a 16S ribosomal RNA sequence of *I. massiliensis* deposited at DSM under number DSM 33460. As described herein, the composition comprises (a) EVs derived from the *E. eligens* strain deposited at DSM under number DSM 33458 or a live strain having all of the identifying characteristics of the *E. eligens* strain deposited at DSM under number DSM 33458; (b) EVs derived from the *I. massiliensis* strain deposited at DSM under number DSM 33460 or a live strain having all of the identifying characteristics of the *I*. *massiliensis* strain deposited at DSM under number DSM 33460; (c) EVs derived from the *P. copri* strain deposited at DSM under number DSM 33457 or a live strain having all of the identifying characteristics of the *P. copri* strain deposited at DSM under number DSM 33457; and/or (d) EVs derived from the *Akkermansia sp.* deposited at DSM under number DSM 33459 or a live strain having all of the identifying characteristics of the *Akkermansia sp.* deposited at DSM under number DSM 33459 either (A) alone; and/or (B) in combination with a culture supernatant derived from one or more of these strains. Only compositions comprising EVs derived from *Akkermansia sp* DSM 33459 as set forth in the claims are within the scope of the present invention. As described herein, the composition comprises (b) EVs derived from a biologically pure strain of *Intestinimonas massiliensis;* and (d) EVs derived from a biologically pure strain of an *Akkermansia sp.,* wherein said *Akkermansia sp.* is not (i) *Akkermansia muciniphila*; or (ii) *Akkermansia glycaniphilia.* Described herein, the composition comprises (b) EVs derived from a bacterial strain having a 16S ribosomal RNA sequence displaying at least 97.0% sequence similarity to a 16S ribosomal RNA sequence of *I. massiliensis* deposited at DSM under number DSM 33460. Described herein, genome-wide average nucleotide identity (gANI) between said *Akkermansia sp.* and (i) *Akkermansia muciniphila;* or (ii) *Akkermansia glycaniphilia* is less than about 95%. In some embodiments of any of the embodiments disclosed herein, the composition comprises (b) EVs derived from the *I. massiliensis* strain deposited at DSM under number DSM 33460 or a live strain having all of the identifying characteristics of the *I. massiliensis* strain deposited at DSM under number DSM 33460; and (d) EVs derived from the *Akkermansia sp.* deposited at DSM under number DSM 33459. Only compositions comprising EVs derived from *Akkermansia sp* DSM 33459 as set forth in the claims are within the scope of the present invention. In some embodiments of any of the embodiments disclosed herein, the composition is formulated for oral administration. In some embodiments of any of the embodiments disclosed herein, the composition is lyophilized or freeze dried. In some embodiments of any of the embodiments disclosed herein, the composition is encapsulated or coated. In some embodiments of any of the embodiments disclosed herein, the composition is a food product, food ingredient, dietary supplement, or medicament. In some embodiments disclosed herein, at least about 1 x 10⁴ CFU/g composition to at least about 1 x 10¹² CFU/g composition of bacteria is present in the composition. In some embodiments disclosed herein, the composition is a probiotic. In some embodiments of any of the embodiments disclosed herein, the composition has been pasteurized or heat treated. In some embodiments of any of the embodiments disclosed herein, the composition is a pharmaceutical composition and further comprises at least one pharmaceutically acceptable carrier and/or excipient.

In other aspects, provided herein is a tablet, prolonged-release capsule, prolonged-release granule, powder, sachet, or gummy comprising any of the compositions (such as probiotic compositions) according to the claims.

In still further aspects, provided herein is a kit comprising (a)(i) any of the compositions (such as probiotic compositions) according to the claims; or (ii) any of the tablet, prolonged-release capsule, prolonged-release granule, powder, sachet, or gummy according to the claims and b) written instructions for administration to a subject.

In yet another aspect, provided herein is a composition, tablet, prolonged-release capsule, prolonged-release granule, powder, sachet, or gummy according to the claims for use in a method for treating and/or preventing one or more obesity related disorders in a subject in need thereof, comprising administering a therapeutically effective amount of any of the compositions according to the claims or any of the tablet, prolonged-release capsule, prolonged-release granule, powder, sachet, or gummy according to the claims to the subject. In some embodiments, the obesity related disorder is one or more disorders selected from the group consisting of obesity, metabolic syndrome, diabetes mellitus, insulin deficiency-related disorders, insulin-resistance related disorders, glucose intolerance, abnormal lipid metabolism, non-alcoholic fatty liver disease, hepatic steatosis, leptin resistance, reduced resistin levels, and/or cardiovascular disease.

Described herein is a method for making a composition comprising combining a biologically pure strain of *Intestinimonas massiliensis* and a biologically pure strain of an *Akkermansia sp.,* wherein said *Akkermansia sp.* is not (i) *Akkermansia muciniphila;* or (ii) *Akkermansia glycaniphilia.* Described herein, the genome-wide average nucleotide identity (gANI) between said *Akkermansia sp.* and (i) *Akkermansia muciniphila;* or (ii) *Akkermansia glycaniphilia* is less than about 95%. In some embodiments of any of the embodiments disclosed herein, the *I. massiliensis* comprises a 16S ribosomal RNA sequence displaying at least 97.0% sequence similarity to a 16S ribosomal RNA sequence of *I. massiliensis* deposited at DSM under number DSM 33460. In some embodiments of any of the embodiments disclosed herein, the *I*. *massiliensis* comprises the *I. massiliensis* strain deposited at DSM under number DSM 33460; and wherein the *Akkermansia sp.* comprises the *Akkermansia sp.* deposited at DSM under number DSM 33459. In some embodiments of any of the embodiments disclosed herein, the method further comprises freeze frying or lyophilizing the composition. Only methods falling within the scope of the claims are within the scope of the present invention.

In other aspects, provided herein is a composition for use in the prevention and/or treatment of one or more obesity-related disorders in a subject in need thereof, comprising any of the compositions (such as probiotic compositions) according to the claims or any of the tablet, prolonged-release capsule, prolonged-release granule, powder, sachet, or gummy according to the claims to the subject. In some embodiments, the obesity related disorder is one or more disorders selected from the group consisting of obesity, metabolic syndrome, diabetes mellitus, insulin deficiency-related disorders, insulin-resistance related disorders, glucose intolerance, abnormal lipid metabolism, non-alcoholic fatty liver disease, hepatic steatosis, leptin resistance, reduced resistin levels, and/or cardiovascular disease.

Each of the aspects and embodiments described herein are capable of being used together, unless excluded either explicitly or clearly from the context of the embodiment or aspect, provided this falls within the scope of the appended claims.

### BRIEF DESCRIPTION OF THE DRAWINGS

**FIG. 1A** depicts differentially abundant fecal 16S rRNA operational taxonomic units (OTUs) measured in lean healthy compared to obese prediabetic individuals and their corresponding association to clinical metabolic markers using Spearman's correlation coefficient analyses. **FIG. 1B** depicts OTUs taxonomically defined as *Intestinimonas, Prevotella, Eubacterium* and *Akkermansia* sp. identified from a clinical study that compared lean healthy individuals to obese prediabetic (elevated BMI, insulin and glucose), indicating their positive association to metabolic health.
**FIG. 2** depicts a phylogenetic tree consists of strain AF3360009 and the strains from class Verrucomicrobiae that were included in Ouwerkerk et al., 2016. The tree was reconstructed with neighbor-joining method with 1000 bootstraps. Numbers represent bootstrap values. The legend bar indicates 5% sequence divergence. *Chlamydia trachomatis* was used as outgroup.
**FIG. 3** depicts scanning electron microscopy images of strain AF3360009. They are oval or elongated with filament like structures when grown on (left) YCFA and mucin compared to (right) YCFA.
**FIG. 4** depicts a graph showing the effect of *Eubacterium eligens, Intestinimonas massiliensis, Prevotella copri, Akkermansia sp. and Intestinimonas massiliensis + Akkermansia sp.* on insulin levels in a DIO mouse model.
**FIG. 5** depicts a graph showing the effect of *Eubacterium eligens, Intestinimonas massiliensis, Prevotella copri, Akkermansia sp. and Intestinimonas massiliensis + Akkermansia sp.* on leptin levels in a DIO mouse model.
**FIG. 6A** and **FIG. 6B** depict graphs showing the effect of *Eubacterium eligens, Intestinimonas massiliensis, Prevotella copri, Akkermansia sp. and Intestinimonas massiliensis + Akkermansia sp.* on glucose tolerance in a DIO mouse model.
**FIG. 7** depicts a graph showing the effect of *Eubacterium eligens, Intestinimonas massiliensis, Prevotella copri, Akkermansia sp. and Intestinimonas massiliensis + Akkermansia sp.* on cholesterol levels in a DIO mouse model.
**FIG. 8** depicts a graph showing the effect of *Eubacterium eligens, Intestinimonas massiliensis, Prevotella copri, Akkermansia sp. and Intestinimonas massiliensis + Akkermansia sp.* on resistin levels in a DIO mouse model.
**FIG. 9** depicts an *Akkermansia* gANI dendrogram comparing publicly-available genomes of *A. muciniphila, A. glycaniphilia,* and strain AF3360009.
**FIG. 10** depicts effect of *Intestinimonas massiliensis* and *Akkermansia sp.* (Frozen, Pasteurized and Lyophilized) on body weight in DIO model.
**FIG. 11** depicts effect of *Intestinimonas massiliensis* and *Akkermansia sp.* (Frozen, Pasteurized and Lyophilized) on body fat weight.
**FIG. 12** depicts effect of *Intestinimonas massiliensis* and *Akkermansia sp.* (Frozen, Pasteurized and Lyophilized) on liver weight.
**FIG. 13** depicts effect of *Intestinimonas massiliensis* and *Akkermansia sp.* (Frozen, Pasteurized and Lyophilized) on insulin levels in DIO model.
**FIG. 14** depicts measurement of insulin resistance by HOMA-IR.
**FIG. 15** depicts effect of *Intestinimonas massiliensis* and *Akkermansia sp.* (Frozen, Pasteurized and Lyophilized) on leptin levels in DIO model.
**FIG. 16** depicts effect of *Intestinimonas massiliensis* and *Akkermansia sp.* (Frozen, Pasteurized and Lyophilized) on Plasminogen activator inhibitor-1 (PAI 1) levels in DIO model.
**FIG. 17** depicts effect of *Intestinimonas massiliensis* and *Akkermansia sp.* (Frozen, Pasteurized and Lyophilized) on resistin levels in DIO model.
**FIG. 18** depicts production of SCFA by *Akkermansia* sp. and *I. massiliensis.*
**FIG. 19** depicts sample comparison of CE-TOFMS relative peak areas of Agmatine.
**FIG. 20** depicts removal of extracellar ATP by *Akkermansia* strains

### DETAILED DESCRIPTION

Many previous studies have shown that probiotic bacteria, for example from the genera *Lactobacillus* and *Bifidobacterium,* support the growth of beneficial gut bacteria colonies but it also seems that certain beneficial probiotic strains can also alter host metabolism pathways for the better. Microbial organisms produce bioactive substances that influence carbohydrate and lipid metabolism and modulate both intestinal and systemic inflammatory processes. Thus, there has been increasing interest in identifying nutritional supplements and probiotic foods that are effective for the control of obesity and obesity related disorders.

The inventors of the instant application have surprisingly found that microorganisms outside of the commonly used probiotics *Lactobacillus* and *Bifidobacterium* can successfully alter gut metabolism and ameliorate conditions associated with obesity. These beneficial microorganisms were found to be both enriched in the digestive systems of healthy people of normal weight and were deficient in individuals suffering one or more obesity related disorders. Supplementation of one or more of the beneficial microorganisms to the diets of mice modeling human obesity resulted in substantial improvement on one or more metrics relevant to negative conditions associated with obesity.

### I. Definitions

As used herein, "microorganism" or "microbe" refers to a bacterium, a fungus, a virus, a protozoan, and other microbes or microscopic organisms.

As used here in the term "probiotic" refers to a composition for consumption by animals (*i.e.* as an or as a component of animal feed) that contains viable (*i.e.* live) microorganisms, *i.e.* microorganisms that are capable of living and reproducing that, when administered in adequate amounts, confer a health benefit on a subject (*see* Hill et al. 2014 Nature Revs Gastro & Hep 11, 506-514). A probiotic may comprise one or more (such as any of 1, 2, 3, or 4) of any of the microbial strains described herein. Probiotics are distinguished from bacterial compositions that have been killed, for example, by pasteurization or heat treatment. Administration of non-viable bacterial compositions for the treatment of one or more metabolic disorders is also contemplated in certain embodiments of the methods disclosed herein.

A bacterial "strain" as used herein refers to a bacterium which remains genetically unchanged when grown or multiplied. The multiplicity of identical bacteria is included.

By "at least one strain," is meant a single strain but also mixtures of strains comprising at least two strains of microorganisms. By "a mixture of at least two strains," is meant a mixture of two, three, four, five, six or even more strains. Described herein, in a mixture of strains, the proportions can vary from 1% to 99%. When a mixture comprises more than two strains, the strains can be present in substantially equal proportions in the mixture or in different proportions.

For purposes of this disclosure, a "biologically pure strain" means a strain containing no other bacterial strains in quantities sufficient to interfere with replication of the strain or to be detectable by normal bacteriological techniques. "Isolated" when used in connection with the organisms and cultures described herein includes not only a biologically pure strain, but also any culture of organisms which is grown or maintained other than as it is found in nature. As described herein, the strains are mutants, variants, or derivatives of strains *Eubacterium eligens, Intestinimonas massiliensis, Prevotella copri,* and/or an *Akkermansia sp.,* wherein the *Akkermansia sp.* is not *A. muciniphila* or *A. glycaniphilia* that also provide benefits comparable to that provided by *Eubacterium eligens, Intestinimonas massiliensis, Prevotella copri,* and/or an *Akkermansia sp.,* wherein the *Akkermansia sp.* is not *A. muciniphila* or *A. glycaniphilia.* As described herein the strains are strains having all of the identifying characteristics of strains *Eubacterium eligens, Intestinimonas massiliensis, Prevotella copri,* and/or an *Akkermansia sp.,* wherein *the Akkermansia sp.* is not *A. muciniphila* or *A. glycaniphilia.* Further, each individual strain (*Eubacterium eligens, Intestinimonas massiliensis, Prevotella copri,* and/or an *Akkermansia sp.,* wherein *the Akkermansia sp.* is not *A. muciniphila* or *A. glycaniphilia*) or any combination of these strains can also provide one or more of the benefits described herein. It will also be clear that addition of other microbial strains, carriers, additives, enzymes, yeast, or the like will also provide one or more benefits or improvement of one or more metabolic conditions in a subject and will not constitute a substantially different bacterial strain.

The term "16S rRNA" or "16S ribosomal RNA" means the rRNA constituting the small subunit of prokaryotic ribosomes. In bacteria, this sequence can be used to identify and characterize operational taxonomic units.

The term "sequence identity" or "sequence similarity" as used herein, means that two polynucleotide sequences, a candidate sequence and a reference sequence, are identical (*i.e.* 100% sequence identity) or similar (*i.e.* on a nucleotide-by-nucleotide basis) over the length of the candidate sequence. In comparing a candidate sequence to a reference sequence, the candidate sequence may comprise additions or deletions (*i.e.* gaps) as compared to the reference sequence (which does not comprise additions or deletions) for optimal alignment of the two sequences. Optimal alignment of sequences for determining sequence identity may be conducted using the any number of publicly available local alignment algorithms known in the art such as ALIGN or Megalign (DNASTAR), or by inspection.

The term "percent (%) sequence identity" or "percent (%) sequence similarity," as used herein with respect to a reference sequence is defined as the percentage of nucleotide residues in a candidate sequence that are identical to the residues in the reference polynucleotide sequence after optimal alignment of the sequences and introducing gaps, if necessary, to achieve the maximum percent sequence identity.

As used herein, the term "subject" or "patient" is meant a mammal (e.g., a human). In some embodiments, a subject is suffering from obesity, metabolic syndrome, diabetes mellitus, insulin deficiency-related disorders, insulin-resistance related disorders, glucose intolerance, abnormal lipid metabolism, non-alcoholic fatty liver disease, hepatic steatosis, leptin resistance, reduced resistin levels, and/or cardiovascular disease. In some embodiments, a subject is susceptible to a disease, disorder, or condition. In some embodiments, a subject displays one or more symptoms or characteristics of a disease, disorder or condition. In some embodiments, a subject does not display any symptom or characteristic of a disease, disorder, or condition. In some embodiments, a subject is someone with one or more features characteristic of susceptibility to or risk of a disease, disorder, or condition. In some embodiments, a subject is a patient. In some embodiments, a subject is an individual to whom diagnosis and/or therapy is and/or has been administered.

As used herein, "prevent," "preventing," "prevention" and grammatical variations thereof refers to a method of partially or completely delaying or precluding the onset or recurrence of obesity, metabolic syndrome, diabetes mellitus, insulin deficiency-related disorders, insulin-resistance related disorders, glucose intolerance, abnormal lipid metabolism, non-alcoholic fatty liver disease, hepatic steatosis, leptin resistance, reduced resistin levels, and/or cardiovascular disease) and/or one or more of its attendant symptoms or barring a subject from acquiring or reacquiring a disorder or condition or reducing a subject's risk of acquiring or reacquiring a disorder or condition or one or more of its attendant symptoms.

As used herein, the term "reducing" in relation to a particular trait, characteristic, feature, biological process, or phenomena refers to a decrease in the particular trait, characteristic, feature, biological process, or phenomena. The trait, characteristic, feature, biological process, or phenomena can be decreased by 5%, 10%, 15%, 20%, 25%, 30%, 35%, 40%, 45%, 50%, 55%, 60%, 65%, 70%, 75%, 80%, 85%, 90%, 95%, 100% or greater than 100%.

As used herein "administer" or "administering" is meant the action of introducing one or more compositions comprising one or more microbial strain, to a subject, such as by feeding or consuming orally. The composition containing one or more microbial strains can also be administered in one or more doses. References to methods of administering a composition disclosed herein to a subject, in the description and examples of this description, are to be interpreted as references to compounds, pharmaceutical compositions and medicaments of the present invention for use in those methods.

As used herein, "effective amount" means a quantity of a composition containing one or more microbial strains to improve one or more metrics in subject. Improvement in one or more metrics of an subject (such as, without limitation, any of treating and/or preventing obesity, metabolic syndrome, diabetes mellitus, insulin deficiency-related disorders, insulin-resistance related disorders, glucose intolerance, abnormal lipid metabolism, non-alcoholic fatty liver disease, hepatic steatosis, leptin resistance, reduced resistin levels, and/or cardiovascular disease) can be measured as described herein or by other methods known in the art.

Certain ranges are presented herein with numerical values being preceded by the term "about." The term "about" is used herein to provide literal support for the exact number that it precedes, as well as a number that is near to or approximately the number that the term precedes. In determining whether a number is near to or approximately a specifically recited number, the near or approximating unrecited number can be a number which, in the context in which it is presented, provides the substantial equivalent of the specifically recited number. For example, in connection with a numerical value, the term "about" refers to a range of -10% to +10% of the numerical value, unless the term is otherwise specifically defined in context.

As used herein, the singular terms "a," "an," and "the" include the plural reference unless the context clearly indicates otherwise.

It is further noted that the claims may be drafted to exclude any optional element. As such, this statement is intended to serve as antecedent basis for use of such exclusive terminology as "solely," "only" and the like in connection with the recitation of claim elements or use of a "negative" limitation.

It is also noted that the term "consisting essentially of," as used herein refers to a composition wherein the component(s) after the term is in the presence of other known component(s) in a total amount that is less than 30% by weight of the total composition and do not contribute to or interferes with the actions or activities of the component(s).

It is further noted that the term "comprising," as used herein, means including, but not limited to, the component(s) after the term "comprising." The component(s) after the term "comprising" are required or mandatory, but the composition comprising the component(s) can further include other non-mandatory or optional component(s).

It is also noted that the term "consisting of," as used herein, means including, and limited to, the component(s) after the term "consisting of." The component(s) after the term "consisting of" are therefore required or mandatory, and no other component(s) are present in the composition.

It is intended that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

Unless defined otherwise herein, all technical and scientific terms used herein have the same meaning as commonly understood by one of ordinary skill in the art to which this invention pertains.

Other definitions of terms may appear throughout the specification.

### II. Compositions

### A. Strains

The beneficial microbial-containing compositions disclosed herein can be used as supplements, food additives, and therapeutics for administration to subjects when under periods of physiologic stress (disease state, metabolic state, etc.) or as a part of a daily nutritional regimen to prevent disease and facilitate healthy gut metabolism. Probiotics is another term that can be used for these compositions which contain viable microorganisms. The term "viable microorganism" means a microorganism which is metabolically active or able to differentiate. In some embodiments, the beneficial microbial-containing compositions disclosed herein include both viable probiotic products and/or, in particular embodiments, compositions that include non-viable bacteria (such as heat-treated or pasteurized compositions).

The strains provided herein include a biologically pure strain of *Eubacterium eligens,* a biologically pure strain of *Intestinimonas massiliensis;* a biologically pure strain of *Prevotella copri* and a biologically pure strain of an *Akkermansia sp.,* wherein said *Akkermansia sp.* is not *Akkermansia muciniphila;* or *Akkermansia glycaniphilia.*

The *E. eligens* strain, *I. massiliensis* strain, the *P*. copri strain, and the *Akkermansia sp.* strain were deposited on March 4, 2020 at the German Collection of Microorganisms and Cell Cultures GmbH (DSM), Inhoffenstraβe 7B, 38124 Braunschweig, GERMANY and given accession numbers DSM 33458, DSM 33460, DSM 33457, and DSM 33459, respectively. The deposits were made under the provisions of the Budapest Treaty on the International Recognition of the Deposit of Microorganisms for the Purposes of Patent Procedure.

The microbial-containing compositions according to the claims (such as probiotic compositions) comprise *Akkermansia* strain DSM 33459. The microbial-containing compositions according to the claims (such as probiotic compositions) can include those that contain one or more strains (such as any of about 1, 2, 3, 4, 5, 6, 7, or 8 or more strains) of *Eubacterium eligens* (such as *E. eligens* strain DSM 33458). *E. eligens* is a gram-positive bacterium in the family Eubacteriaceae, characterized by a rigid cell wall.

The microbial-containing compositions according to the claims (such as probiotic compositions) comprise *Akkermansia* strain DSM 33459. The microbial-containing compositions (such as probiotic compositions) according to the claims can include those that contain one or more strains (such as any of about 1, 2, 3, 4, 5, 6, 7, or 8 or more strains) of *Intestinimonas massiliensis* (such as *I. massiliensis* strain DSM 33460). *I*. *massiliensis* is a nonmotile, gram-negative rod with a mean diameter of 0.5 µm and 1.8 µm in length, without spore-forming activity (Durand et al., 2017, New Microbes New Infect., 15: 1-2). In some embodiments, the beneficial microbial-containing composition according to the claims includes both *I*. *massiliensis* and *Akkermansia* strain DSM 33459. Additionally, when cultured or administered together, one or more *I*. *massiliensis* strain (such as *I*. *massiliensis* strain DSM 33460) and *Akkermansia* strain DSM 33459 exhibit one or more physiological or metabolic properties that individually cultured *I*. *massiliensis* strain (such as *I. massiliensis* strain DSM 33460) and *Akkermansia* strain DSM 33459 lacks. These properties can include, without limitation, changes in the amount and/or type of organic acid produced, change in metabolic profile, and/or a change in the composition of media in which the bacteria are cultured together.

The beneficial microbial-containing compositions (such as probiotic compositions) according to the claims can include one or more *I. massiliensis* strain having a 16S ribosomal RNA sequence displaying at least about 97.0% sequence similarity (such as any of about 97%, 97.5%, 98%, 98.5%, 99%, 99.5%, or 100% sequence similarity) to a 16S ribosomal RNA sequence comprising SEQ ID NO:2.

### B. Formulations

Only compositions falling under the scope of the claims fall within the scope of protection of the present invention. Any reference to administering a composition or formulation herein should be taken as the composition for use in said administering. Generally, the microbial-containing compositions (such as probiotic compositions) disclosed herein comprise bacteria, such as one or more bacterial strains. Described herein, the composition is formulated in freeze-dried or lyophilized form. For example, the microbial-containing compositions can comprise granules or gelatin capsules, for example hard gelatin capsules, comprising a bacterial strain disclosed herein.

In some embodiments, the microbial-containing compositions according to the claims comprise lyophilized bacteria. Lyophilization of bacteria is a well-established procedure in the art. Alternatively, the microbial-containing compositions according to the claims can comprise a live, active bacterial culture.

In some embodiments, any of the microbial-containing compositions according to the claims is encapsulated to enable delivery of the bacterial strain to the intestine. Encapsulation protects the composition from degradation until delivery at the target location through, for example, rapturing with chemical or physical stimuli such as pressure, enzymatic activity, or physical disintegration, which may be triggered by changes in pH. Any appropriate encapsulation method may be used. Exemplary encapsulation techniques include entrapment within a porous matrix, attachment or adsorption on solid carrier surfaces, self-aggregation by flocculation or with cross-linking agents, and mechanical containment behind a microporous membrane or a microcapsule.

The microbial-containing compositions disclosed herein can be administered orally and may be in the form of a tablet, capsule or powder. Other ingredients (such as vitamin C or minerals, for example), may be included as oxygen scavengers and prebiotic substrates to improve the delivery and/or partial or total colonization and survival *in vivo.* Alternatively, the microbial-containing compositions (such as probiotic compositions) disclosed herein can be administered orally as a food or nutritional product, such as milk or whey based fermented dairy product, or as a pharmaceutical product.

The microbial-containing compositions disclosed herein can be formulated as a probiotic. Alternatively, the microbial-containing compositions disclosed herein can be formulated as a non-viable bacterial compositions, such as a pasteurized or heat-treated bacterial composition.

A microbial-containing composition disclosed herein includes a therapeutically effective amount of a bacterial strain disclosed herein. A therapeutically effective amount of a bacterial strain is sufficient to exert a beneficial effect upon a patient A therapeutically effective amount of a bacterial strain may be sufficient to result in delivery to and/or partial or total colonisation of the subject's intestine.

A suitable daily dose of the bacteria, for example for an adult human, may be from about 1 x 10³ to about 1 X 10¹¹ colony forming units (CFU); for example, from about 1 x 10⁷ to about 1 x 10¹⁰ CFU; in another example from about 1 x 10⁶ to about 1 x 10¹⁰ CFU; in another example from about 1 x 10⁷ to about 1 x 10¹¹ CFU; in another example from about 1 x 10⁸ to about 1 x 10¹⁰ CFU; in another example from about 1 x 10⁸ to about 1 x 10¹¹ CFU. In certain embodiments, the dose of the bacteria is at least 10⁹ cells per day, such as at least 10¹⁰, at least 10¹¹ or at least 10¹² cells per day.

In certain embodiments, the microbial-containing composition contains the bacterial strain in an amount of from about 1 x 10⁶ to about 1 x 10¹¹ CFU/g, respect to the weight of the composition; for example, from about 1 x 10⁸ to about 1 x 10¹⁰ CFU/g. The dose may be, for example, 1 g, 3g, 5g, and 10 g.

In certain embodiments, any of the microbial-containing compositions disclosed herein is administered at a dose of between 500mg and 1000mg, between 600mg and 900mg, between 700mg and 800mg, between 500mg and 750mg or between 750mg and 1000mg. In certain embodiments, the lyophilized bacteria in any of the microbial-containing compositions disclosed herein is administered at a dose of between 500mg and 100mg, between 600mg and 900mg, between 700mg and 800mg, between 500mg and 750mg or between 750mg and 1000mg.

Typically, a probiotic, is optionally combined with at least one suitable prebiotic compound. A prebiotic compound is usually a non-digestible carbohydrate such as an oligo- or polysaccharide, or a sugar alcohol, which is not degraded or absorbed in the upper digestive tract Known prebiotics include commercial products such as inulin and transgalacto-oligosaccharides.

In certain embodiments, a probiotic composition according to the claims is formulated to include a prebiotic compound in an amount of from about 1 to about 30% by weight respect to the total weight composition, (*e.g.* from 5 to 20% by weight). Carbohydrates may be selected from the group consisting of: fructo- oligosaccharides (or FOS), short-chain fructo-ol igosaccharides, inulin, isomalt- oligosaccharides, pectins, xylo-oligosaccharides (or XOS), chitosan-oligosaccharides (or COS), human milk oligosaccharides, beta- glucans, gum arabic modified and resistant starches, polydextrose, D-tagatose, acacia fibers, carob, oats, and citrus fibers. In one aspect, the prebiotics are the short-chain fructo-oligosaccharides (for simplicity shown herein below as FOSs-c.c); said FOSs-c.c. are not digestible carbohydrates, generally obtained by the conversion of the beet sugar and including a saccharose molecule to which three glucose molecules are bonded. In some embodiments, any of the probiotics according to the claims can be formulated with additional probiotics derived from the genera *Lactobacillus* and *Bifidobacterium* (such as *B. lactis* B420).

The microbial-containing composition disclosed herein can further comprise pharmaceutically acceptable excipients or carriers. Acceptable carriers or diluents for therapeutic use are well known in the pharmaceutical art. Examples of suitable carriers include, without limitation, lactose, starch, glucose, methyl cellulose, magnesium stearate, mannitol, sorbitol and the like. Examples of suitable diluents include, without limitation, ethanol, glycerol and water. The choice of pharmaceutical carrier, excipient or diluent can be selected with regard to the intended route of administration and standard pharmaceutical practice. The pharmaceutical compositions may comprise as, or in addition to, the carrier, excipient or diluent any suitable binders, lubricants, suspending agents, coating agents (such as a gastric-resistant enteric coating agent that does not dissolve or degrade until reaching the small or large intestine), or solubilizing agents. Examples of suitable binders include, without limitation, starch, gelatin, natural sugars such as glucose, anhydrous lactose, free-flow lactose, beta-lactose, corn sweeteners, natural and synthetic gums, such as acacia, tragacanth or sodium alginate, carboxymethyl cellulose and polyethylene glycol. Examples of suitable lubricants include, without limitation, sodium oleate, sodium stearate, magnesium stearate, sodium benzoate, sodium acetate, sodium chloride and the like. Preservatives, stabilizers, dyes and even flavoring agents may be provided in the pharmaceutical composition. Examples of preservatives include, without limitation, sodium benzoate, sorbic acid and esters of p-hydroxybenzoic acid. Antioxidants and suspending agents may be also used.

The microbial-containing composition disclosed herein can be formulated as a food product. For example, a food product may provide nutritional benefit in addition to the therapeutic effect of the invention, such as in a nutritional supplement. Similarly, a food product may be formulated to enhance the taste of the composition of the invention or to make the composition more attractive to consume by being more similar to a common food item, rather than to a pharmaceutical composition. In certain embodiments, the microbial-containing composition is formulated as a milk-based product. The term "milk-based product," as used herein, means any liquid or semi-solid milk- or whey- based product having a varying fat content. The milk- based product can be, *e.g.,* cow's milk, goat's milk, sheep's milk, skimmed milk, whole milk, milk recombined from powdered milk and whey without any processing, or a processed product, such as yoghurt, curdled milk, curd, sour milk, sour whole milk, butter milk and other sour milk products. Another important group includes milk beverages, such as whey beverages, fermented milks, condensed milks, infant or baby milks; flavored milks, ice cream; milk-containing food such as sweets.

The microbial-containing compositions according to the claims (such as probiotic compositions) comprise *Akkermansia* strain DSM 33459. In certain embodiments, the microbial-containing compositions according to the claims contain a single bacterial strain or species and do not contain any other bacterial strains or species. Such compositions may comprise only *de minimis* or biologically irrelevant amounts of other bacterial strains or species. Such compositions may be a culture that is substantially free from other species of organism. In certain embodiments, the compositions of the invention consist of 1, 2, 3, 4, 5, 6, 7, 8, 9, 10, 11, 12, 13, 14, 15 or 16 bacterial strains or species. In certain embodiments, the compositions consist of from 1 to 10, such as from 1 to 5 bacterial strains or species.

The microbial-containing composition for use in accordance with the methods disclosed herein may or may not require marketing approval.

In some cases, the lyophilized bacterial strain is reconstituted prior to administration. In some cases, the reconstitution is by use of a diluent described herein.

The microbial-containing compositions disclosed herein can comprise pharmaceutically acceptable excipients, diluents or carriers.

In certain embodiments, provided herein is a pharmaceutical composition according to the claims comprising: *Akkermansia* strain DSM 33459; and a pharmaceutically acceptable excipient, carrier or diluent; wherein the bacterial strain is in an amount sufficient to treat a disorder when administered to a subject in need thereof; and wherein the disorder is selected from the group consisting of obesity, metabolic syndrome, diabetes mellitus, insulin deficiency-related disorders, insulin-resistance related disorders, glucose intolerance, abnormal lipid metabolism, non-alcoholic fatty liver disease, hepatic steatosis, leptin resistance, reduced resistin levels, and/or cardiovascular disease.

In certain embodiments, the invention provides the above pharmaceutical composition according to the claims, comprising a carrier selected from the group consisting of lactose, starch, glucose, methyl cellulose, magnesium stearate, mannitol and sorbitol.

In certain embodiments, the invention provides the above pharmaceutical composition according to the claims, comprising a diluent selected from the group consisting of ethanol, glycerol and water.

In certain embodiments, the invention provides the above pharmaceutical composition according to the claims, comprising an excipient selected from the group consisting of starch, gelatin, glucose, anhydrous lactose, free-flow lactose, beta-lactose, corn sweetener, acacia, tragacanth, sodium alginate, carboxymethyl cellulose, polyethylene glycol, sodium oleate, sodium stearate, magnesium stearate, sodium benzoate, sodium acetate and sodium chloride.

In certain embodiments, the invention provides the above pharmaceutical composition according to the claims, further comprising at least one of a preservative, an antioxidant and a stabilizer.

In certain embodiments, the invention provides the above pharmaceutical composition according to the claims, comprising a preservative selected from the group consisting of sodium benzoate, sorbic acid and esters of p- hydroxybenzoic acid.

In certain embodiments, the invention provides the above pharmaceutical composition according to the claims, wherein said bacterial strain is lyophilized.

In certain embodiments, the above pharmaceutical composition according to the claims, wherein when the composition is stored in a sealed container at about 4°C or about 25°C and the container is placed in an atmosphere having 50% relative humidity, at least 80%, 70%, 60%, 50%, 40%, 30%, 20%, or 10% of the bacterial strain as measured in colony forming units, remains after a period of at least about 1 month, 3 months, 6 months, 1 year, 1.5 years, 2 years, 2.5 years or 3 years.

The bacterial strains disclosed herein can be cultured using standard microbiology techniques such as those described in the Examples section or that are well known in the art.

In additional embodiments, one or more of the bacterial strains disclosed herein can be formulated as compositions (such as pharmaceutical compositions) comprising bacterial extracellular vesicles (EVs). As used herein, the term "extracellular vesicle" or "EV" or refers to a composition derived from a bacterium that comprises bacterial lipids, and bacterial proteins and/or bacterial nucleic acids and/or carbohydrate moieties contained in a nanoparticle. These EVs may contain 1, 2, 3, 4, 5, 10, or more than 10 different lipid species. EVs may contain 1, 2, 3, 4, 5, 10, or more than 10 different protein species. EVs may contain 1, 2, 3, 4, 5, 10, or more than 10 different nucleic acid species. EVs may contain 1, 2, 3, 4, 5, 10, or more than 10 different carbohydrate species. As used herein, the term "purified EV composition" or "EV composition" refer to a preparation that includes EVs that have been separated from at least one associated substance found in a source material (e.g. separated from at least one other bacterial component) or any material associated with the EVs in any process used to produce the preparation. It also refers to a composition that has been significantly enriched or concentrated. In some embodiments the EVs are concentrated by 2-fold, 3-fold, 4-fold, 5-fold, 10-fold, 100-fold, 1000-fold, 10,000-fold or more than 10,000-fold.

The EVs described herein can be prepared using any method known in the art. In some embodiments, the EVs are prepared without an EV purification step. For example, in some embodiments, bacteria comprising the EVs according to the claims are killed using a method that leaves the bacterial EVs intact and the resulting bacterial components, including the EVs, are used in the methods and compositions described herein. In some embodiments, the bacteria are killed using an antibiotic (*e.g.,* using an antibiotic described herein). In some embodiments, the bacteria are killed using UV irradiation. In some embodiments, the EVs according to the claims are purified from one or more other bacterial components. Methods for purifying EVs from bacteria are known in the art. In some embodiments EVs are prepared from bacterial cultures using methods described in S. Bin Park, et al. PLoS ONE. 6(3):el7629 (2011) or G. Norheim, et al. PLoS ONE. 10(9): e0134353 (2015). In some embodiments, the bacteria are cultured to high optical density and then centrifuged to pellet bacteria (*e.g.,* at 10,000 x g for 30 min at 4°C). In some embodiments, the culture supernatants are then passed through filter to exclude intact bacterial cells (*e.g.,* a 0.22 µm filter). In some embodiments, filtered supernatants are centrifuged to pellet bacterial EVs (*e.g.,* at 100,000-150,000 x g for 1-3 hours at 4°C). In some embodiments, the EVs are further purified by resuspending the resulting EV pellets (*e.g.,* in PBS), and applying the resuspended EVs to sucrose gradient (*e.g.,* a 30-60% discontinuous sucrose gradient), followed by centrifugation (*e.g*., at 200,000 x g for 20 hours at 4°C). EV bands can be collected, washed with (*e.g.,* with PBS), and centrifuged to pellet the EVs (*e.g.,* at 150,000 x g for 3 hours at 4°C). The purified EVs can be stored, for example, at - 80°C until use. In some embodiments, the EVs are further purified by treatment with DNase and/or proteinase K.

For example, in some embodiments, cultures of bacteria according to the claims can be centrifuged at 11,000 x g for 20-40 min at 4°C to pellet bacteria. Culture supernatants may be passed through a 0.22 µm filter to exclude intact bacterial cells. Filtered supernatants may then be concentrated using methods that may include, but are not limited to, ammonium sulfate precipitation, ultracentrifugation, or filtration. For example, for ammonium sulfate precipitation, 1.5-3 M ammonium sulfate can be added to filtered supernatant slowly, while stirring at 4°C. Precipitations can be incubated at 4°C for 8-48 hours and then centrifuged at 11,000 x g for 20-40 min at 4°C. The resulting pellets contain bacterial EVs and other debris.

Using ultracentrifugation, filtered supernatants can be centrifuged at 100,000-200,000 x g for 1-16 hours at 4°C. The pellet of this centrifugation contains bacterial EVs and other debris. In some embodiments, using a filtration technique, such as through the use of an Amicon Ultra spin filter or by tangential flow filtration, supernatants can be filtered so as to retain species of molecular weight > 50 or 100 kDa.

Alternatively, EVs can be obtained from bacterial cultures continuously during growth, or at selected time points during growth, by connecting a bioreactor to an alternating tangential flow (ATF) system (*e.g*., XCell ATF from Repligen). The ATF system retains intact cells (>0.22 µm) in the bioreactor, and allows smaller components (*e.g*., EVs, free proteins) to pass through a filter for collection. For example, the system may be configured so that the <0.22 um filtrate is then passed through a second filter of 100 kDa, allowing species such as EVs between 0.22 µm and 100 kDa to be collected, and species smaller than 100 kDa to be pumped back into the bioreactor. Alternatively, the system may be configured to allow for medium in the bioreactor to be replenished and/or modified during growth of the culture. EVs collected by this method may be further purified and/or concentrated by ultracentrifugation or filtration as described above for filtered supernatants.

EVs obtained by methods provided herein may be further purified by size-based column chromatography, by affinity chromatography, and by gradient ultracentrifugation, using methods that may include, but are not limited to, use of a sucrose gradient or Optiprep gradient. Briefly, using a sucrose gradient method, if ammonium sulfate precipitation or ultracentrifugation were used to concentrate the filtered supernatants, pellets are resuspended in 60% sucrose, 30 mM Tris, pH 8.0. If filtration was used to concentrate the filtered supernatant, the concentrate is buffer exchanged into 60% sucrose, 30 mM Tris, pH 8.0, using an Amicon Ultra column. Samples are applied to a 35-60% discontinuous sucrose gradient and centrifuged at 200,000 x g for 3-24 hours at 4°C. Briefly, using an Optiprep gradient method, if ammonium sulfate precipitation or ultracentrifugation were used to concentrate the filtered supernatants, pellets are resuspended in 35% Optiprep in PBS. In some embodiments, if filtration was used to concentrate the filtered supernatant, the concentrate is diluted using 60% Optiprep to a final concentration of 35% Optiprep. Samples are applied to a 35-60% discontinuous sucrose gradient and centrifuged at 200,000 x g for 3-24 hours at 4°C.

In some embodiments, to confirm sterility and isolation of the EV preparations, EVs are serially diluted onto agar medium used for routine culture of the bacteria being tested and incubated using routine conditions. Non-sterile preparations are passed through a 0.22 µm filter to exclude intact cells. To further increase purity, isolated EVs may be DNase or proteinase K treated.

### III. Methods

### A. Methods for Treating or Preventing Disease

Any references to methods of treatment by therapy or methods of administering a composition disclosed herein to a subject, in the description and examples of this description, are to be interpreted as references to compounds, pharmaceutical compositions and medicaments of the present invention for use in those methods. Further provided herein are methods for treating and/or preventing one or more obesity related disorders including obesity, metabolic syndrome, diabetes mellitus, insulin deficiency-related disorders, insulin-resistance related disorders, glucose intolerance, abnormal lipid metabolism, non-alcoholic fatty liver disease, hepatic steatosis, leptin resistance, reduced resistin levels, and/or cardiovascular disease in a subject in need thereof, comprising administering to the subject a therapeutically effective amount of any of the microbial-containing and/or EV-containing compositions according to the claims.

The body mass index (BMI) (calculated as weight in kilograms divided by the square of height in meters) is the most commonly accepted measurement for overweight and/or obesity. In adults, a BMI exceeding 25 is considered overweight, while obesity is defined as a BMI of 30 or more, with a BMI of 35 or more considered as serious co-morbidity and a BMI of 40 or more considered morbid obesity. For the purposes of this invention, "obesity" shall mean a BMI of 30 or more.

One out of every five overweight people is affected by the "metabolic syndrome". Metabolic syndrome is one of the fastest growing obesity-related health concerns in the United States and is characterized by a cluster of health problems including obesity, hypertension, abnormal lipid levels, and high blood sugar. According to the Centers for Disease Control and Prevention (CDC), the metabolic syndrome affects almost one quarter (22 percent) of the American population-an estimated 47 million people. The assemblage of problems characterized as comprising the metabolic syndrome can increase a patient's risk for developing more serious health problems, such as diabetes, heart disease, and stroke.

Overweight and obese people have an increased incidence of heart disease, and thus fall victim to heart attack, congestive heart failure, sudden cardiac death, angina, and abnormal heart rhythm more often than those that maintain a healthy body mass index. Obesity often increases the risk of heart disease because of its negative effect on blood lipid levels, which increase in obese patients and then, in turn, increase triglyceride levels and decrease high-density lipoprotein-which is also known as HDL. People with an excessive amount of body fat have higher levels of triglycerides and low-density lipoprotein-which is also known as LDL or "bad cholesterol"-as well as lower levels of HDL cholesterol in the blood. This combination creates optimal conditions for developing atherosclerotic heart disease.

Being overweight or obese increases the risk of developing high blood pressure. Hypertension, or high blood pressure, greatly raises the risk of heart attack, stroke, and kidney failure. In fact, blood pressure rises as body weight increases. Losing even 10 pounds can lower blood pressure-and losing weight has the biggest effect on those who are overweight and already have hypertension.

Obesity is associated with the development of diabetes. More than 80 percent of people with type 2 diabetes, the most common form of the disease, are obese or overweight. Type 2 diabetes develops when either there is impaired insulin production by the pancreas in the setting of insulin resistance in the tissues and organs in the body. As obesity diminishes insulin's ability to control blood sugar (glucose), there is an increased risk of developing diabetes because the body begins overproducing insulin to regulate blood sugar levels. Over time, the body is no longer able to keep blood sugar levels in the normal range. Eventually the inability to achieve healthy blood sugar balance results in the development of type 2 diabetes. Furthermore, obesity complicates the management and treatment of type 2 diabetes by increasing insulin resistance and glucose intolerance, which makes drug treatment for the disease less effective. In many cases, a reduction of body weight to a normal range normalizes blood glucose and restores insulin sensitivity.

Childhood obesity is also a major public health problem, particularly in Western countries. Children 2-18 years of age are considered obese if the BMI is greater than the 95th percentile. Despite policies targeted at reducing its prevalence, childhood obesity has more than doubled in children and tripled in adolescents in the past 30 years. As with adults, obesity in childhood causes hypertension, dyslipidemia (abnormal lipid metabolism), chronic inflammation, increased blood clotting tendency, endothelial dysfunction, and hyperinsulinemia. This clustering of cardiovascular disease risk factors has been identified in children as young as 5 years of age.

The methods disclosed herein are directed to the prevention, inhibition and treatment of obesity-related disorders. An "obesity-related disorder" as used herein, is obesity, metabolic syndrome, diabetes mellitus, insulin deficiency-related disorders, insulin-resistance related disorders, glucose intolerance, abnormal lipid metabolism, non-alcoholic fatty liver disease, hepatic steatosis, leptin resistance, reduced resistin levels, and/or cardiovascular disease. "Obesity" and "obese" as used herein, refers to class I obesity, class II obesity, class III obesity and pre-obesity (*e.g.,* being "over-weight") as defined by the World Health Organization.

Decreased body fat is expected to provide various primary and/or secondary benefits in a subject (*e.g.,* in a subject diagnosed with a complication associated with obesity, such as an obesity related disorder) such as, for example, an increased insulin responsiveness or decreased glucose intolerance (*e.g.,* in a subject diagnosed with Type II diabetes mellitus); a reduction in elevated blood pressure; a reduction in elevated cholesterol levels and/or LDLs and/or VLDLs; a reduction (or a reduced risk or progression) of cardiovascular disease (including ischemic heart disease, arterial vascular disease, angina, myocardial infarction, and/or stroke), migraines, congestive heart failure, deep vein thrombosis, pulmonary embolism, gall stones, gastroesophagael reflux disease, obstructive sleep apnea, obesity hypoventilation syndrome, asthma, gout, poor mobility, back pain, erectile dysfunction, urinary incontinence, liver injury (*e.g.,* fatty liver disease, liver cirrhosis, alcoholic cirrhosis, endotoxin-mediated liver injury), chronic renal failure, leptin resistance, and elevated resistin levels.

Methods of treatment are excluded from patentability under Article 53(c) EPC and not within the scope of protection. Described herein, the disclosure relates to a method comprising administering to a subject an effective amount of any of the microbial-containing and/or EV-containing compositions (such as probiotic compositions) disclosed herein to reduce obesity. As described herein, the subject's obesity decreases by any of about 10%, 15%, 20%, 25%, 30%, 35%, 40%, 45%, 50%, 55%, 60%, 65%, 70%, 75%, 80%, 85%, 90%, 95%, or 100%, inclusive of all values falling in between these percentages, relative to obese subjects who are not administered one or more of the microbial-containing and/or EV-containing compositions disclosed herein. Reduction in obesity can be measured by any method known in the art, such as reduction in BMI.

Methods of treatment are excluded from patentability under Article 53(c) EPC and not within the scope of protection. Described herein, the disclosure relates to a method comprising administering to a subject an effective amount of any of the microbial-containing and/or EV-containing compositions (such as probiotic compositions) disclosed herein to reduce one or more of metabolic syndrome, diabetes (including type II diabetes), insulin resistance, and/or glucose intolerance. As described herein, the rate of one or more of metabolic syndrome, diabetes (including type II diabetes), insulin resistance, and/or glucose intolerance decreases by any of about 10%, 15%, 20%, 25%, 30%, 35%, 40%, 45%, 50%, 55%, 60%, 65%, 70%, 75%, 80%, 85%, 90%, 95%, or 100%, inclusive of all values falling in between these percentages, relative to subjects diagnosed with one or more of these conditions who are not administered one or more of the microbial-containing and/or EV-containing compositions disclosed herein. Reduction in one or more of metabolic syndrome, diabetes (including type II diabetes), insulin resistance, and/or glucose intolerance can be determined by any means known in the art, such as blood glucose measurement and determination of A1C.

Methods of treatment are excluded from patentability under Article 53(c) EPC and not within the scope of protection. Described herein, the disclosure relates to a method comprising administering to a subject an effective amount of any of the microbial-containing and/or EV-containing compositions (such as probiotic compositions) disclosed herein to treat abnormal lipid metabolism, non-alcoholic fatty liver disease, and/or hepatic steatosis. As described herein, incidence of the hepatic disorder decreases by any of about 10%, 15%, 20%, 25%, 30%, 35%, 40%, 45%, 50%, 55%, 60%, 65%, 70%, 75%, 80%, 85%, 90%, 95%, or 100%, inclusive of all values falling in between these percentages, relative to subjects with hepatic disorders who are not administered one or more of the microbial-containing and/or EV-containing compositions disclosed herein. Reduction in one or more hepatic disorders can be determined by any means known in the art.

Methods of treatment are excluded from patentability under Article 53(c) EPC and not within the scope of protection. Described herein, the disclosure relates to a method comprising administering to a subject an effective amount of any of the microbial-containing and/or EV-containing compositions (such as probiotic compositions) disclosed herein to treat leptin resistance and/or reduced resistin levels. As described herein, leptin resistance decreases and/or resistin levels increase by any of about 10%, 15%, 20%, 25%, 30%, 35%, 40%, 45%, 50%, 55%, 60%, 65%, 70%, 75%, 80%, 85%, 90%, 95%, 100%, 105%, or 110%, inclusive of all values falling in between these percentages, relative to subjects with leptin resistance and/or reduced resistin levels who are not administered one or more of the microbial-containing and/or EV-containing compositions disclosed herein. Leptin resistance and/or reduced resistin levels can be determined by any means known in the art.

Methods of treatment are excluded from patentability under Article 53(c) EPC and not within the scope of protection. Described herein, the disclosure relates to a method comprising administering to a subject an effective amount of any of the microbial-containing and/or EV-containing compositions (such as probiotic compositions) disclosed herein to treat one or more disorders associated with cardiovascular disease (including, without limitation, ischemic heart disease, arterial vascular disease, angina, myocardial infarction, and/or stroke). As described herein, incidence of the hepatic disorder decreases by any of about 10%, 15%, 20%, 25%, 30%, 35%, 40%, 45%, 50%, 55%, 60%, 65%, 70%, 75%, 80%, 85%, 90%, 95%, or 100%, inclusive of all values falling in between these percentages, relative to subjects with one or more disorders associated with cardiovascular disease who are not administered one or more of the microbial-containing and/or EV-containing compositions disclosed herein. Reduction in one or more disorders associated with cardiovascular disease can be determined by any means known in the art.

### B. Methods for Preparing a Microbial Composition

Also provided herein are methods for preparing a microbial-containing and/or EV-containing composition (such as probiotic composition) according to the claims comprising combining a biologically pure strain of *Intestinimonas massiliensis* and a biologically pure strain of *Akkermansia* strain DSM 33459. The *I*. *massiliensis* has the 16S ribosomal RNA sequence of *I. massiliensis* deposited at DSM under number DSM 33460.

Additionally, the methods for preparing the composition can further include lyophilizing or freeze drying the microbial composition. The method can additionally include a further step of packaging the feed additive composition for storage or transport.

### C. Administration

Any references to methods of administering a composition disclosed herein to a subject, in the description and examples of this description, are to be interpreted as references to compounds, pharmaceutical compositions and medicaments of the present invention for use in those methods.

Preferably, the microbial-containing and/or EV-containing compositions disclosed herein are to be administered to the gastrointestinal tract in order to enable delivery to and/or partial or total colonisation of the intestine with the bacterial strain of the invention. Generally, the compositions of the invention are administered orally, but they may be administered rectally, intranasally, or via buccal or sublingual routes.

In certain embodiments, the microbial-containing and/or EV-containing compositions according to the claims may be administered as a foam, as a spray or a gel.

In certain embodiments, the microbial-containing and/or EV-containing compositions according to the claims of the invention may be administered as a suppository, such as a rectal suppository, for example in the form of a theobroma oil (cocoa butter), synthetic hard fat (*e.g.* suppocire, witepsol), glycero-gelatin, polyethylene glycol, or soap glycerin composition.

In certain embodiments, the microbial-containing and/or EV-containing compositions according to the claims are administered to the gastrointestinal tract via a tube, such as a nasogastric tube, orogastric tube, gastric tube, jejunostomy tube (J tube), percutaneous endoscopic gastrostomy (PEG), or a port, such as a chest wall port that provides access to tire stomach, jejunum and other suitable access ports.

The microbial-containing and/or EV-containing compositions disclosed herein may be administered once, or they may be administered sequentially as part of a treatment regimen. In certain embodiments, the compositions of the invention are to be administered daily.

In certain embodiments of the invention, use of the microbial-containing and/or EV-containing compositions according to the claims in a treatment according to methods according to the claims is accompanied by assessment of the subject's gut microbiota. Treatment may be repeated if delivery of and/or partial or total colonization with the strain of the invention is not achieved such that efficacy is not observed, or treatment may be ceased if delivery and/or partial or total colonization is successful, and efficacy is observed. The composition as described herein can be administered to a pregnant animal, for example a mammal such as a human in order to potentially prevent a condition from developing in her child in utero and/or after it is born.

The microbial-containing and/or EV-containing compositions disclosed herein can be administered as a food product, such as a nutritional supplement.

Generally, the microbial-containing and/or EV-containing compositions disclosed herein are for the treatment of human subjects, although they may be used to treat animals including monogastric mammals such as poultry, pigs, cats, dogs, horses or rabbits or multigastric animals such as ruminants. The compositions described herein may be useful for enhancing the growth and performance of animals. If administered to animals, oral gavage may be used.

### IV. Kits

Further provided herein are kits containing one or more of microbial strains and/or EVs derived from one or more of the microbial strains disclosed herein. Only the kits according to the claims are within the scope of the present invention. The kits can include one or more of (such as any of 1, 2, 3, or 4,) strains and/or EVs derived from one or more of the microbial strains provided herein, provided they include strain Akkermansia DSM 33459 or EVs from this strain. In one embodiment, the kit can include *Akkermansia* strain DSM 33459 and *I. massiliensis* strain DSM 33460.

The invention can be further understood by reference to the following examples, which are provided by way of illustration and are not meant to be limiting.

### EXAMPLES

### Example 1: Isolation of strains

**Isolation of *Intestinimonas massiliensis:*** A clinical fecal sample with a higher population of *Intestinimonas massiliensis,* as determined by 16S community analysis of all clinical fecal samples, was used for enrichment protocol, all work performed under anaerobic conditions. Sample was diluted 1:100 in Basal Bicarbonate Buffered Medium, a total of 25ml inoculated media was sealed in a 50ml glass vial. Vial was incubated under anaerobic conditions at 37°C. Approximately 5ml of culture was withdrawn each day dispense into 4 tubes, 2 for DNA extraction and 2 for strain isolation. Samples were taken on days 4, 5, 6, 7, 8, and 11 after the start of the enrichment. DNA was extracted from a sample for each day, and 16S community analysis sequencing was performed to determine the bacterial populations. It was determined that the day 11 sample had the highest percentage of *Intestinimonas massiliensis,* therefore one of the strain isolation samples from day 11 was used to dilute and plate onto Basal Bicarbonate Buffer medium agar plates. Basal Bicarbonate Buffered Media: 0.53g/L sodium phosphate, dibasic, 0.41g/L potassium phosphate, monobasic, 0.3g/L ammonium chloride, 0.11g/L calcium chloride, 0.1g/L magnesium sulfate heptahydrate, 0.3g/L sodium chloride, 4g/L sodium bicarbonate, 0.48g/L sodium sulfide hydrate, 5X Wolfe's trace minerals, 1X standard vitamin solution, 80mM lactate, 80mM acetate.

**Isolation of *Prevotella copri:*** The *Prevotella copri* strain was isolated by plating diluted fecal material directly on BHIB agar plates which incubated anaerobically for 24 hours. BHIB: Brain Heart Infusion agar supplemented with 10% Sheep Blood. (purchased commercially, BD 221843) For growth in liquid broth, BHIS is used. BHIS: Brain Heart Infusion supplemented with yeast extract, Vitamin K1 and hemin.

**Isolation of *Akkermanisa sp.:*** The *Akkermansia* strain was isolated from clinical fecal sample by plating diluted fecal material directly on YCFA medium containing mucin at 10g/L.

**Isolation of *Eubacterium eligens:*** The *E. eligens* strain was isolated by plating diluted fecal material directly on BHIB agar plates which incubated anaerobically for 24 hours. BHIB: Brain Heart Infusion agar supplemented with 10% Sheep Blood. (purchased commercially, BD 221843) For growth in liquid broth, BHIS is used. BHIS: Brain Heart Infusion supplemented with yeast extract, Vitamin K1 and hemin.

**Genome Sequencing:** All strains went through the same method to provide genome sequence. Strains were grown on an agar plate, either BHIB or YCFA. Growth was removed from the agar plate using a large loop, carefully not removing agar. The amount of growth dictated the number of wells which was processed for DNA extraction. For 1 well, the growth was resuspended in 750µl of the first solution utilized in the DNA extraction kit, PowerMag Bead Solution. If there was sufficient growth to expand to multiple wells, then additional volume was used. Cells were resuspended, and the resuspended cells dispensed to the desired number of wells. The DNA extraction protocol is followed using the kit instructions. For elution, slightly less elution buffer was utilized per well to obtain higher DNA concentrations. After completion of the DNA extraction, like wells were combined. DNA concentrations were determined using the Quant-It PicoGreen DSDNA Assay kit from Invitrogen.

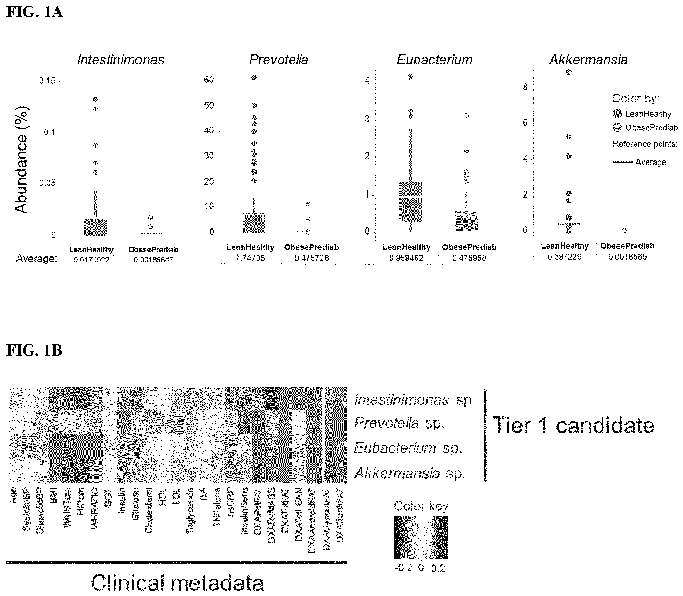

Candidates were isolated from fecal samples of healthy donors and identified by whole genome sequencing. Ranking by statistical analyses including correlation analysis values (insulin, BMI, glucose, DXATotFAT), percent abundance, and number of lean samples carrying the candidates was performed. Candidates were selected based on the correlation analysis and availability of isolation. Four of these candidates were selected for further study **(****FIG. 1A** and

### FIG. 1B).

### Example 2: Identification of a new species of Akkermansia

All work performed in an anaerobic chamber using a mixed gas of N2/CO2/H2 (85/10/5 %), unless otherwise stated.

Strain AF33600009 was isolated during a second isolation round which followed a general round of isolation, with targets being any of the top candidates. The clinical samples selected for this round of isolation were shown to have a higher abundance of the top candidates, based on the previously analyzed 16S community analysis. This strain was isolated from fecal sample F015V3. The isolation method used in this round was selection on YCFA medium containing mucin at 10g/L.

Aliquots used in the isolation round were previously made of fecal material from sample F015V3 mixed with glycerol so that the final glycerol concentration was 25%. These aliquots were stored at -80°C until needed. One aliquot was removed from the freezer, placed in the anaerobic chamber, and allowed to thaw at room temperature for approximately 10 minutes. All work was performed in an anaerobic chamber, unless otherwise stated. A measured portion was removed and diluted serially in YCFA broth without mucin. 100µl aliquots were plated from the 10⁻⁴, 10⁻⁵ and 10⁻⁶ dilutions onto YCFA + mucin agar, in omni trays. Bacterial cells were spread using about a dozen sterile glass beads to spread the dilution aliquot evenly onto the agar surface. Plates were incubated at 37°C for approximately 72 hours in anaerobic boxes with sachets to create an anaerobic environment.

De-oxygenated growth medium, YCFA + mucin at 10g/L, was dispensed into 1 ml deep well plates, 350µl per well. Single colonies were picked and inoculated into the plates with the pre-dispensed medium, 1 colony per well. Plates were covered with a breathable cover allowing for gas exchange. Plates were incubated at 37°C for approximately 216 hours. The cultures were gently mixed using the 96 well head Integra pipettor. An aliquot of culture taken for 16S PCR analysis, and the remainder of the culture mixed with sterile, de-oxygenated 50% glycerol + 1g/L L-Cysteine, so that the final glycerol concentration was 25%. These cultures pipetted to appropriate long-term storage vials, stored at -80°C.

**16S Identification:** The aliquot of cell culture for PCR was diluted approximately 1:100 with sterile water. This dilution into water was used as the template in a 16S PCR reaction. PCR primers used to amplify the 16S gene were: 8F: AGA GTT TGA TYM TGG CTC and 1492R: CGG TTA CCT TGT TAC GAC TT. PCR reaction conditions and Thermocycler setup were standard for polymerase Q5. An aliquot of the 16S PCR reactions were run on a gel to confirm the presence of a 16S PCR product of expected size. An aliquot of the 16S PCR reactions underwent enzymatic purification using the ExoSAP-IT Express for PCR Cleanup Kit. This sample was then sent for sanger sequencing using an outside third-party vendor. The 16C Primer used most frequently for 16S sanger sequencing was 515F: GTG CCA GCM GCC GCG GTA A.

The 16S sequences were then compared against the 16S amplicon sequences of the list of top candidates. These results revealed that the closest matching candidate was *Akkermansia muciniphila.* The vial corresponding to the well containing the desired strain was removed from the freezer and placed into the anaerobic chamber, so that a small portion of the frozen culture could be removed from the vial and streaked onto a YCFA + mucin at 10g/L agar plate. This plate was incubated at 37°C in an anaerobic box with sachets until there was sufficient growth to make a frozen stock and extract DNA.

**Genome of strain AF33600009:** The DNA extraction kit used was the Qiagen MagAttract^{®} PowerSoil^{®} DNA KF (King Fisher) Kit. Growth which had been recently streaked was scrapped off a YCFA + mucin at 10g/L agar plate, and cells were resuspended in the first solution from the DNA extraction kit. Cells were gently resuspended to break up cell clumps, by gently pipetting. This cell resuspension was evenly distributed to multiple wells of the PowerMag Bead Plate. The number of wells was determined by the amount and density of cell the cell suspension. The manufacturer's protocol was then followed for DNA extraction. After completion of DNA extraction, like wells were combined into one DNA sample, and the DNA concentration was determined using the Invitrogen Quant-iT PicoGreen dsDNA quantitation kit. DNA was then sent for whole genome sequencing.

Sequencing libraries were prepared with the Nextera Flex kit (Illumina) and sequenced on MiSeq (Illumina) in paired read 2x150nt. The genome sequencing data was assembled using an in-house pipeline. In brief, reads were filtered and trimmed based on quality then corrected using BFC (Li, 2015). The corrected reads were assembled using the SPAdes assembler (Bankevich et al., 2012) with kmer length option of "31,55,77,99,121". The assembly was corrected with Pilon (Walker et al., 2014). After assembly, the Opening Reading Frames (ORFs) were predicted and annotated by Prokka (Seemann, 2014). 16S rRNA genes were predicted by Barrnap and the closest species identification by RDP pairwise alignment tool (Fish et al., 2013).

The draft genome of strain AF33600009 is comprised of 31 contigs with N50 of 331,405 bp and 125x coverage. The genome size is 3.19 Mb, which is larger than the genome sizes of type strains of the other two *Akkermansia* species: *A. muciniphila* Muc^{T} (2.66 Mbp) and A. *glycaniphila* Pyt^{T} (3.07 Mb). The G+C content of the genomic DNA is 57.7%. Since currently only two species were known in genus *Akkermansia,* a phylogenetic tree was reconstructed with these three strains and some strains from class Verrucomicrobiae that were included in the publication describing A. *glycaniphila* (Ouwerkerk et al., 2016).

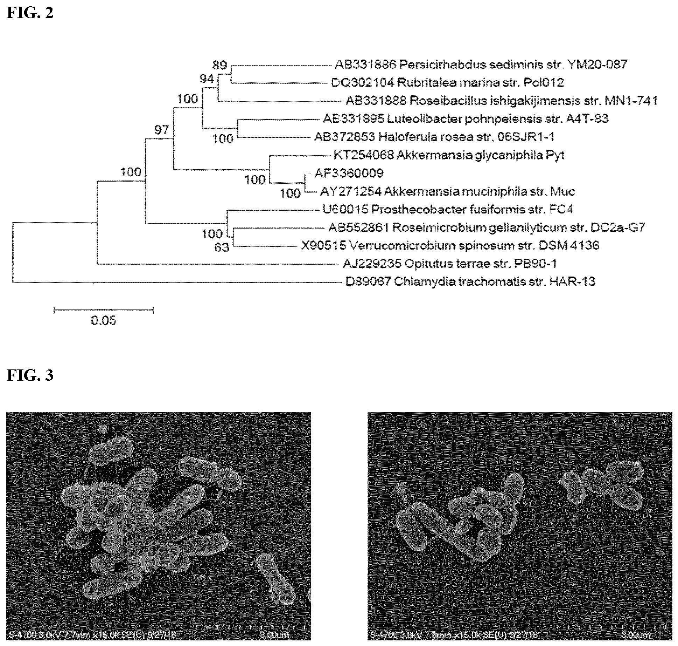

The phylogenetic analysis indicates strain AF33600009 is a member of genus *Akkermansia* with *A. muciniphila* Muc^{T} being its closest relative **(****FIG. 2****).** The genome-wide average nucleotide identity (gANI) between strain AF33600009 and *A. muciniphila* Muc^{T} is 87.58%, and only 70.17% between strain AF33600009 and *A. glycaniphila* Pyt^{T}. Based on the gANI values below the species boundary cutoff of 95% (Goris et al., 2007), strain AF33600009 is proposed as a novel species within genus *Akkermansia.*

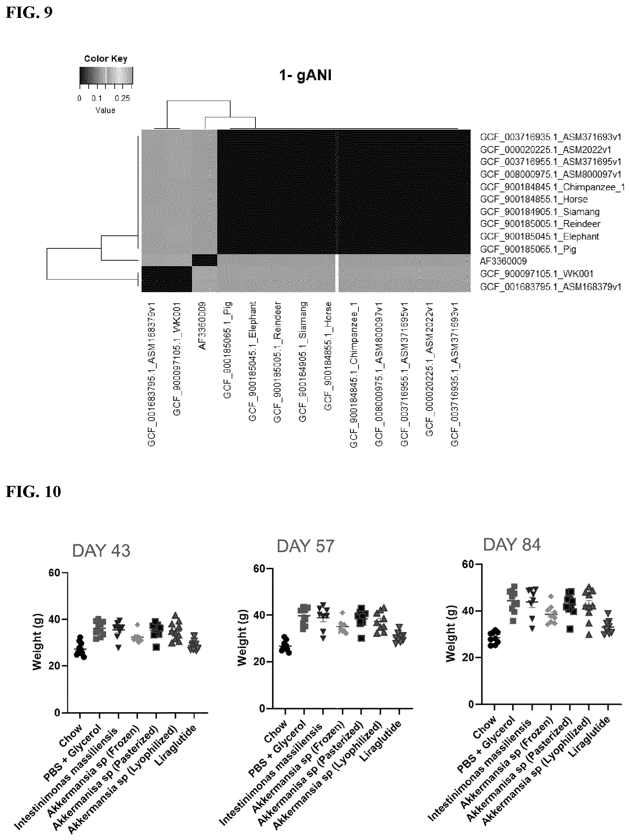

An *Akkermansia* gANI dendrogram was generated using a number of publicly available genomes closest to *A. muciniphila* type strain genome GCF_000020225.1 along with two *A. glycaniphilia* publicly-available genomes and the genome of strain AF33600009 **(****FIG. 9****).** All the genomes from *A. muciniphila* clustered together, the two *A. glycaniphilia* publicly-available genomes clustered together, while AF3360009 formed a separate cluster distinct from the other two species.

A fatty acid methyl esters (FAME) analysis (Welch, 1991. Applications of cellular fatty-acid analysis. Clin. Microbiol. Rev.4:422-438) of cellular fatty acids was performed by Microbial ID Inc (DE, USA), where strain AF33600009 and *A*. *muciniphila* ATCC strain BAA835 underwent standard sample preparation by growing on BHIA to extract the fatty acid methyl esters for identification. Samples are then loaded onto the gas chromatograph for analysis. Using the Sherlock^{®} pattern recognition software, a sample FAME profile was generated. The samples were then compared to determine the similarity. As shown in **Table 1,** the FAME profile showed significant difference between strain AF3360009 and ATCC strain BAA835.

**Table 1: Comparative FAME characteristics of the strains BAA835 and AF3360009.**

| Fatty Acid | AF3360009 | BAA835 |
|---|---|---|
| | | |
| 10:00 | 0.11 | |
| 13:00 | 0.4 | |
| 14:00 iso | 6.27 | 2.32 |
| 14:00 | 0.44 | 2 |
| 14:00 DMA | 1.92 | |
| 15:0 iso | 1.65 | 7.55 |
| 15:00 antiso | 51.62 | 29.73 |
| 15:00 | 9.71 | 0.69 |
| 15:0 3OH | 8.44 | 0.9 |
| 16:0 iso | 3.75 | 4.08 |
| 16:1 w7c | 0.18 | |
| 16:00 | 2.19 | 20.38 |
| 16:0 OH | 0.45 | |
| 17:0 iso | 0.3 | 0.98 |
| 17:0 anteiso | 2.25 | 7.84 |
| 17:00 | 3.11 | |
| 17:0 anteiso 3OH | 0.56 | |
| 17:0 3OH | 2.08 | |
| 18:1 w9c | 0.51 | 18.18 |
| 18:00 | 0.49 | 1.2 |
| un 18.199 | | 0.7 |
| 18:1 w7c DMA | | 0.67 |
| Summed Feature 5 | 2.38 | |
| Summed Feature 6 | 0.55 | |
| Summed Feature 9 | 0.47 | |
| Summed Feature 11 | 0.18 | |
| Summed Feature 10 | | 1.95 |
| Summed Feature 12 | | 0.84 |

Cells of strain AF33600009 were oval or elongated. Elongation was observed more when the cells were grown on medium with mucin. Cells were more in aggregates and formed more filaments when grown on YCFA+mucin medium compared to YCFA only **(****FIG. 3****).**

### Example 3: Mouse model for evaluating the efficacy of candidates

Only experiments with compositions comprising *Akkermansia sp.* DSM 33459 are according to the invention.

A Diet Induced Obesity (DIO) mouse model was employed for evaluating the efficacy of the candidates: *Eubacterium eligens; Intestinimonas massiliensis, Prevotella copri* and the *Akkermansia sp.* described in Example 2.

*E. eligens; I*. *massiliensis, P. copri and Akkermansia sp.* were evaluated for their efficacy in improving the metabolic disorders in an DIO mice model. For animal study, Group 1 animals were maintained on PMI Nutrition International Certified Rodent Chow No. 5 CR4 upon arrival. Group 2 - 9 animals were maintained on Research Diet D12492. Animals were single housed in polycarbonate cages which contained appropriate bedding. Animals were distributed to the treatment groups as mentioned in the **Table 2** on study day -1, in such a way as to generate cohorts with no significant differences with respect to body weight and non-fasted blood glucose based on measurements from Day -1.

**Table 2: Experimental design**

| **Group No.** | **Test Material** | **Strain** | **Dose Level (mg/kg bw or cfu)** | **Dose Volume (µl/mouse)** | **Dose Concentrati on (mg/mL)** | **Number. of Animals (Males)** |
|---|---|---|---|---|---|---|
| 1 | No Tx | C57 | NA | NA | NA | 10 |
| 2 | Vehicle | DIO | 0 | 100 | NA | 10 |
| 3 | *Eubacterium eligens* | | 5x10⁸ | 100 | NA | 10 |
| 4 | *Intestinimonas* | | 1x10⁹ | 100 | NA | 10 |
| 5 | *Prevotella copri* | | 1x10⁹ | 100 | NA | 10 |
| 6 | *Akkermansia* | | 1x10⁹ | 100 | NA | 10 |
| 7 | *Intestinimonas + Akkermansia* | | 5x10⁸ + 5x10⁸ | 100 | NA | 10 |
| 8 | Liraglutide | | 0.2 mg/kg | 5 mL/kg | 0.04 | 10 |
| | | | | | | |

Test articles *Eubacterium eligens, Intestinimonas massiliensis, Prevotella copri, Akkermansia sp.* and *Intestinimonas massiliensis + Akkermansia sp.* were prepared daily and administered within one hour of formulation. The Vehicle (Group 2) was administered once daily via oral gavage from Days 1 to 36. The dose volume for each animal was 100 µl. Test Articles (Groups 2 - 7) were administered once daily via oral gavage from Days 1 to 36. The dose volume for each animal was 100 µl. Each dose was administered using a syringe with attached gavage cannula.

The Control Article (Group 8) was administered to the appropriate animals via subcutaneous injection into the interscapular area once daily from Days 1 to 36. The dose volume for each animal was based on the most recent body weight measurement. Each dose was administered using a using a syringe/needle within the demarcated area. The first day of dosing was designated as Day 1.

Study parameters included mortality/moribundity checks, daily observations, body weight measurements, food consumption, fecal samples, blood glucose measurements, Oral Glucose Tolerance Test, qNMR assessments, Cytokine assessments, and Clinical Chemistry parameters. Blood samples were taken at designated time points throughout the dosing phase and on the scheduled euthanasia day for biomarker evaluation.

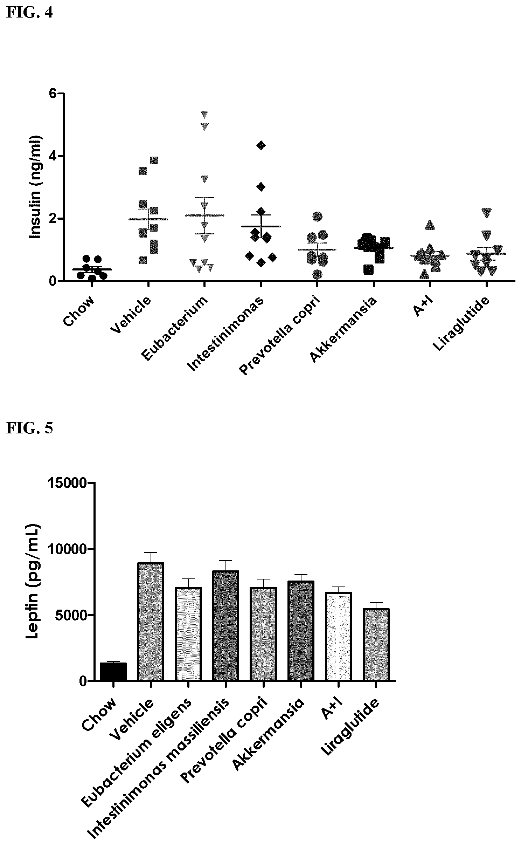

Insulin levels were measured in the serum obtained from mice from different groups. *Intestinimonas massiliensis* gavaged group showed 12% decrease in insulin levels compared to vehicle control. *Prevotella copri, Akkermansia sp.* and *Intestinimonas massiliensis + Akkermansia sp* gavaged group showed improvement in insulin levels by 50%, 50% and 64%, respectively **(****FIG. 4****).**

Leptin levels were measured in the serum obtained from mice from different groups. *Eubacterium eligens* gavaged group showed 21% decrease in insulin levels compared to vehicle control. *Prevotella copri, Akkermansia sp.* and *Intestinimonas massiliensis + Akkermansia sp* gavaged group showed improvement in leptin levels by 20%, 15% and 25%, respectively **(****FIG. 5****).**

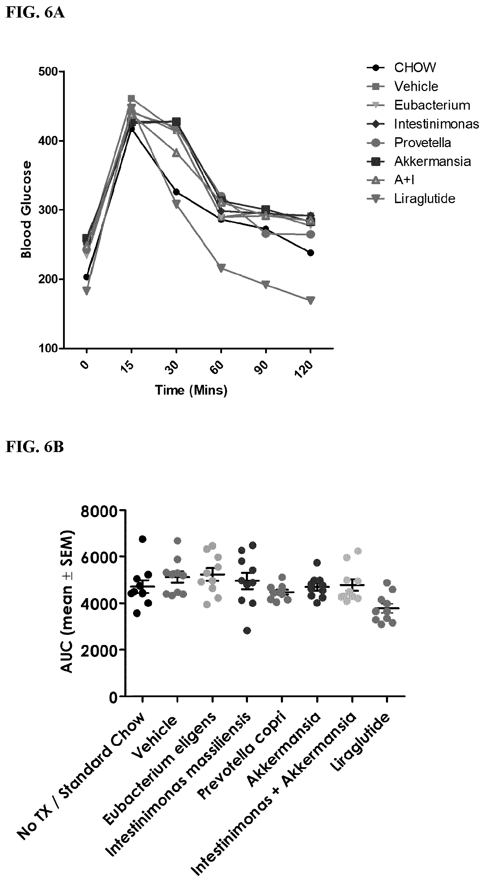

Following a 2 hour fast, all mice were dosed intraperitoneally with 2.0 g/kg glucose (10 mL/kg). Blood glucose was checked via tail snip using a hand-held glucometer at the following times relative to glucose dose: 0 (pre-glucose dose, 15, 30, 60, 90, and 120 min. *Prevotella copri, Akkermansia sp.* and *Intestinimonas massiliensis + Akkermansia sp* gavaged group showed improvement in glucose tolerance by 9.5 %, 10% and 8.5%, respectively **(****FIG. 6A** and **FIG. 6B****).**

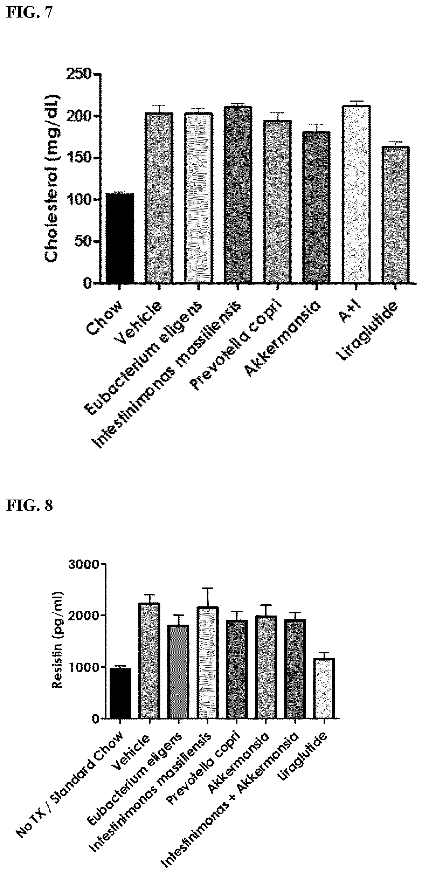

Cholesterol levels were measured in the serum obtained from mice from different groups. *Akkermansia sp* gavaged group showed improvement in cholesterol levels by 11% **(****FIG. 7****).**

Resistin levels were measured in the serum obtained from mice from different groups. *Eubacterium eligens* gavaged group showed 19 % decrease in resistin levels compared to vehicle control. *Prevotella copri, Akkermansia sp. and Intestinimonas massiliensis + Akkermansia sp* gavaged group showed improvement in insulin levels by 14%, 11% and 14% **(****FIG. 8****).**

### Example 4: Mouse model for evaluating formulations

Only experiments with compositions comprising *Akkermansia sp.* DSM 33459 are according to the invention.

*Intestinimonas massiliensis* and *Akkermansia sp.* (Frozen, Pasteurized and Lyophilized) were evaluated for their efficacy in improving the metabolic disorders in an DIO mice model. For animal study, Group 1 animals were maintained on PMI Nutrition International Certified Rodent Chow No. 5 CR4 upon arrival. Group 2 - 7 animals were maintained on Research Diet D12492. Animals were single housed in polycarbonate cages which contained appropriate bedding. Animals were distributed to the treatment groups as mentioned in the **Table 3** on study day -1, in such a way as to generate cohorts with no significant differences with respect to body weight and non-fasted blood glucose based on measurements from Day -1.

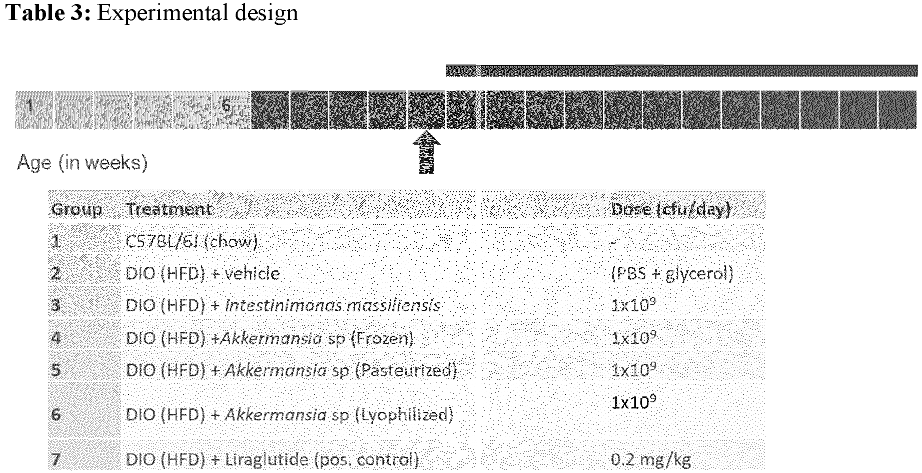

Test articles *Intestinimonas massiliensis* and *Akkermansia sp.* (Frozen, Pasteurized and Lyophilized) were prepared daily and administered within one hour of formulation. The Vehicle (Group 2) was administered once daily via oral gavage from Days 1 to 84. The dose volume for each animal was 100 µl. Test Articles (Groups 2 - 6) were administered once daily via oral gavage from Days 1 to 84. The dose volume for each animal was 100 µl. Each dose was administered using a syringe with attached gavage cannula.

The Control Article (Group 7) was administered to the appropriate animals via subcutaneous injection into the interscapular area once daily from Days 1 to 84. The dose volume for each animal was based on the most recent body weight measurement. Each dose was administered using a using a syringe/needle within the demarcated area. The first day of dosing was designated as Day 1.

Study parameters included mortality/moribundity checks, daily observations, body weight measurements, food consumption, fecal samples, blood glucose measurements, qNMR assessments, and Clinical Chemistry parameters.

Body weight were measured every week during the study. As shown in **FIG. 10****,** vehicle control showed a significant increase in the body weight compared to the chow-only group. The group receiving *Akkermansia* sp in frozen format showed 9% to 14% improvement in body weight from day 43 to day 84.

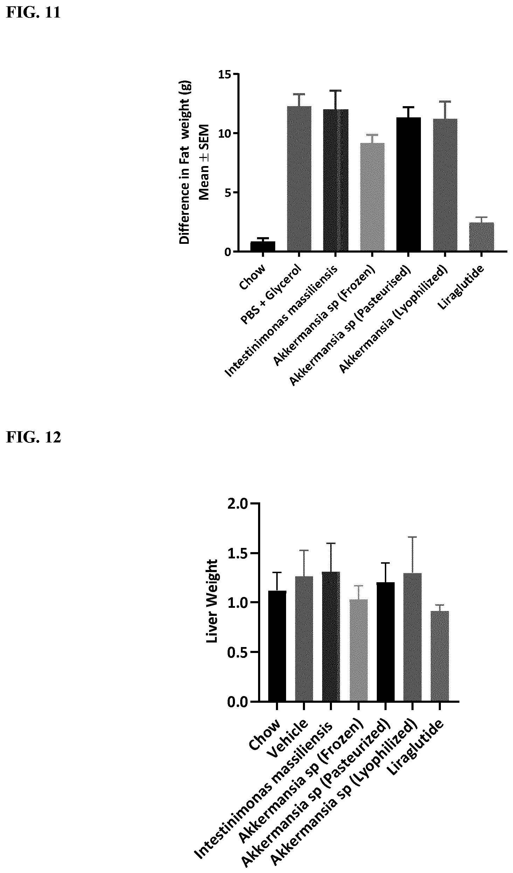

Body composition analysis was done by qNMR (Bruker NMR LF90II). Body composition was determined at the start (days -2 and -1) and at the end of the study (Days 83 and 84). As shown in **FIG. 11**, vehicle control showed significant fat accumulation in the animals compared to the chow-only group. The group receiving *Akkermansia* sp. in frozen format showed 25% less fat accumulation compared to control. *Akkermansia* sp in lyophilized and pasteurized formats also showed less fat accumulation (5.5% and 5%, respectively).

Liver weight was determined at the end of the study (Day 84). As shown in **FIG. 12****,** vehicle control showed increase in the liver weight in the animals compared to the chow-only group. The group receiving *Akkermansia sp.* in frozen format showed 37% less fat accumulation of fat compared to control

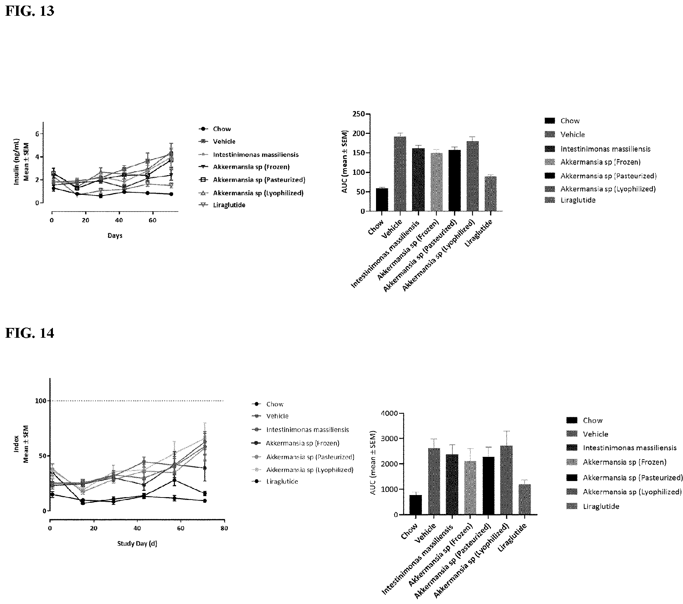

Insulin levels were measured in the serum obtained from mice from different groups. Measurements were done once every 2 weeks during the study. Area under the curve was calculated based on the values obtained during the study. As shown in **FIG. 13****,** vehicle control showed increase in the insulin levels in the animals compared to chow. The group receiving *I*. *massiliensis* showed 17% decrease and group receiving *Akkermansia sp.* in frozen format showed 22% decrease in insulin levels compared to control. *Akkermansia sp.* in lyophilized and pasteurized formats also showed decreases in insulin levels (5% and 17%, respectively).

Insulin resistance was measured by HOMA-IR. Measurement was done based on the fasting glucose and insulin values. Area under the curve was calculated based on the values obtained during the study. As shown in **FIG. 14****,** vehicle control showed increase in the insulin resistance in the animals compared to chow. Group receiving *I. massiliensis* showed 10 % decrease and group receiving *Akkermansia sp.* in frozen format showed 20% decrease in insulin levels compared to control. *Akkermansia sp.* in pasteurized format also showed decreases in insulin levels of 12%.

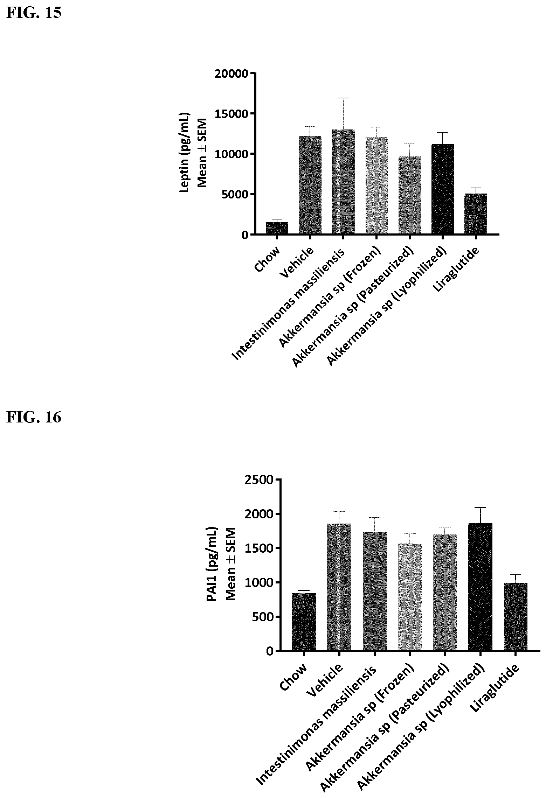

Leptin levels were measured in the serum obtained from mice from different groups. As shown in **FIG. 15*****,** Akkermansia sp.* (pasteurized format) gavaged group showed improvement in leptin levels by 21%. *Akkermansia* sp. in lyophilized format also showed decreases in leptin levels (7%).

PAI1 levels were measured in the serum obtained from mice from different groups.. As shown in **FIG. 16**, *Akkermansia sp* (frozen format) gavaged group showed improvement in PAI1 levels by 15%. *Akkermansia* sp. (pasteurized format) showed improvement in PAI1 levels by 8%.

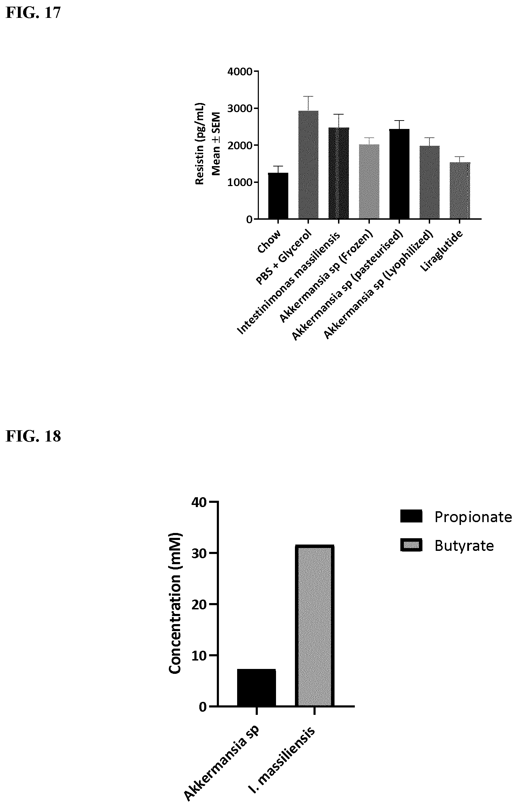

Resistin levels were measured in the serum obtained from mice from different groups. As shown in **FIG. 17****,** the *I. massiliensis* gavaged group showed 16% decrease in resistin levels compared to vehicle control. *Akkermansia sp.* in frozen format showed 31%, pasteurized showed 17% and lyophilized format showed 32% improvement in resistin levels, respectively.

Production of SCFA by *Akkermansia sp* and *I. massiliensis* was analyzed by growing the bacteria in RCM for 72 hrs. Supernatants were collected, filtered and SCFA was detected by HPLC. *Akkermansia sp.* showed production of propionate and *I. massilinesis* showed production of butyrate in the media **(****FIG. 18****).** It has been shown that propionate and butyrate both plays a critical role in modulating host metabolic health (Chambers et al 2018, Ríos-Covián et al 2016).

In this model, *Akkermansia sp* (frozen format) showed significant improvements in body weight, fat accumulation, liver weight, Insulin resistance and resistin levels. *Akkermansia sp.* in the pasteurized form showed similar, but less pronounced activity suggesting that administration of live bacteria may be preferred. While *Akkermansia sp.* when administered in a lyophilized format was not as effective as frozen format, it still showed improvement in resistin and leptin levels, and some improvements in fat accumulation as well as insulin levels. Without being bound to theory, the reason why the lyophilized format was not as efficacious is likely due to insufficient hydration time of the cells when given in this format. As the transient time in mice is 3-4 hrs and it is possible that in this model lyophilized format did not have enough for sufficient hydration and transcriptional activity to drive the same efficacy.

### Example 5: Metabolic analysis of Akkermansia sp strain AF3360009

For comparison of metabolic capabilities of the strain *Akkermansia sp.* and *Akkermansia muciniphila* type strain *BAA835* growth in YCFAC media. YCFAC media was inoculated with 1% overnight culture and supernatants were collected after 24 hrs of growth. Cells were separated by centrifugation at 10,000rpm for 5 mins and then filtered by 0.2 µM filter.

Cell-free supernatants were harvested and analyzed by CE-TOF-MS. Cationic conditions: Samples was injected using 50mbar, 10 sec onto Fused Silica Capillary (i.d. 50 µm x 0 cm) on an Agilent CE-TOF System (Agilent Technologies Inc., Santa Clara, CA, USA). Cationic Buffer solution (1M Formic acid) was used and a CE-voltage of 30kV. Positive mode mass spectrometer conditions: MS Capillary voltage: 4.0V, ESI Positive ionization mode, m/z range 50-1000. Anionic conditions: Samples was injected using 50mbar, 22 sec onto Fused Silica Capillary (i.d. 50µm x 0cm) on an Agilent CE-TOF System (Agilent Technologies Inc.). Anionic Buffer solution (50 mM Ammonium acetate pH 7.5) was used and a CE-voltage of 30kV. Negative mode mass spectrometer conditions: MS Capillary voltage: 3.5V, ESI Negative ionization mode, m/z range 50-1000.

The metabolite Agmatine (N-(4-aminobutyl)guanidine) was detected in cationic mode at retention time 4.23mins at m/z 131.130 m.u, and the identification was performed against a known standard. The reported amounts are peak areas.

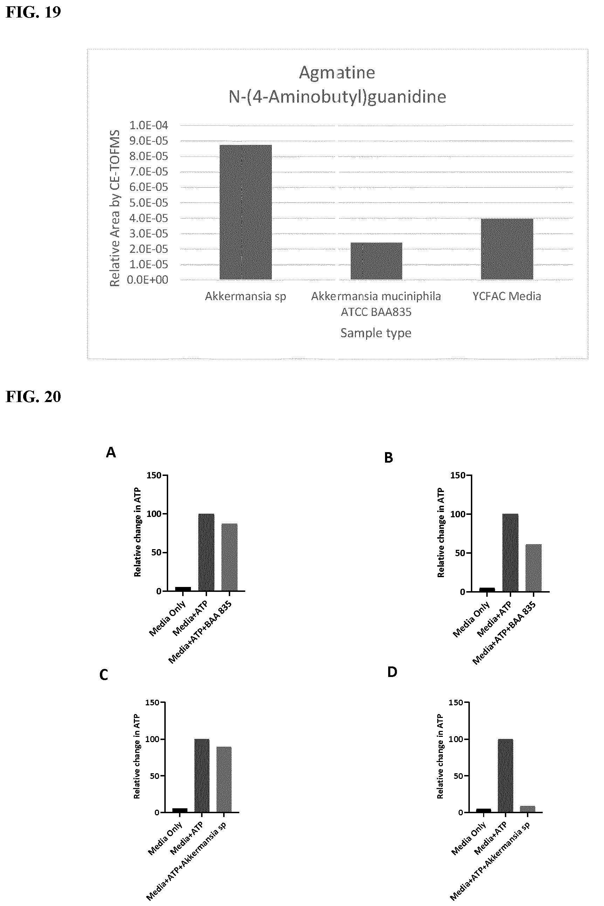

Agmatine is metabolized from arginine by the enzyme arginine decarboxylase EC 4.1.1.19 (Piletz et al 2013, Taksande et al 2016). As shown in **FIG. 19****,** the level of agmatine is higher *in Akkermansia* sp (8.7E10-5) compared to *Akkermansia municiniphila* ATCC BAA 835 (2.4E10-5) and in the YCFAC growth media (4.0E10-5). The measured difference between the two strains and the media level indicates that *Akkermansia* sp. is producing agmatine from arginine while *Akkermansia muciniphila* ATCC BA835 is consuming agmatine.

Extracellular ATP has been shown to induce inflammation (Cauwels et al 2014). *Akkermansia sp.* and *Akkermansia muciniphila BAA835* were evaluated for their capabilities to remove ATP from the growth media. YCFAC media with mucin (10g/l) was inoculated with 1% overnight culture and spiked with 1 mM ATP. supernatants were collected just after inoculation and after 8 hrs of growth. ATP levels were measured by using standard techniques. Cells were separated by centrifugation at 10,000rpm for 5 mins and then filtered by 0.2 µM filter. Cell-free supernatants were harvested and analyzed for the ATP levels.

ATP amounts were detected in the supernatants for *Akkermansia muciniphila* ATCC BAA 835 at time 0 **(****FIG. 20A****)** and after 8 hrs of growth **(****FIG. 20B****).** Similarly ATP was estimated in the supernatants from *Akkermansia* sp at time 0 **(****FIG. 20C****)** and after 8 hrs of growth **(****FIG. 20D****).** *Akkermansia* sp was able to remove ATP more efficiently as compared to *A. muciniphila* ATCC BAA 835.

Using a genome wide comparison, a possible operon coding for enzyme machinery involved in the synthesis of Vitamin B12 is present in the *Akkermansia sp.* and is absent in the type strain *A*. *muciniphila* ATCC BAA835 **(Table 4)**. The ability to synthesize this important cofactor suggests broader metabolic capabilities of *Akkermansia* sp. compared to the type strain.

**Table 4: Genome comparison between Akkermansia sp and A. muciniphila ATCC BAA835 for the presence of Corrin ring biosynthesis gene cluster.**

| Gene | Akkermansia sp | BAA835 | ECnumber | product |
|---|---|---|---|---|
| cbiA | 1 | 0 | 6.3.5.11 | Cobyrinate a,c-diamide synthase |
| cbiC | 1 | 0 | 5.4.99.60 | Cobalt-precorrin-8 methylmutase |
| cbiD | 1 | 0 | 2.1.1.195 | Cobalt-precorrin-5B C(1)-methyltransferase |
| cbiET | 1 | 0 | unknownEC | Cobalamin biosynthesis bifunctional protein CbiET |
| cbiF | 1 | 0 | 2.1.1.271 | Cobalt-precorrin-4 C(11)-methyltransferase |
| cbiKp | 1 | 0 | 4.99.1.3 | Sirohydrochlorin cobaltochelatase CbiKP |
| cbiL | 1 | 0 | 2.1.1.151 | Cobalt-precorrin-2 C(20)-methyltransferase |
| chiA1 | 1 | 0 | 3.2.1.14 | Chitinase A1 |
| cobO | 1 | 0 | 2.5.1.17 | Cob(I)yrinic acid a,c-diamide adenosyltransferase |

### REFERENCES

Bankevich A, et al. SPAdes: a new genome assembly algorithm and its applications to single-cell sequencing. J Comput Biol. 2012;19(5):455-77.
Collado, M. C., et al. (2007). Intestinal integrity and Akkermansia muciniphila, a mucindegrading member of the intestinal microbiota present in infants, adults, and the elderly. Appl Environ Microbiol 73, 7767-7770.
Costello, E. K., et al. (2010). Postprandial remodeling of the gut microbiota in Burmese pythons. ISME J 4, 1375- 1385.
Chun J., et al. (2018). Proposed minimal standards for the use of genome data for the taxonomy of prokaryotes. Int. J. Syst. Evol. Microbiol. 68 461-466. 10.1099/ijsem.0.002516
Fish JA, et al. FunGene: thefunctional gene pipeline and repository. Front Microbiol. 2013;4:291. doi:10.3389/fmicb.2013.00291.
Goris, J., et al. (2007). DNA-DNA hybridization values and their relationship to whole-genome sequence similarities. Int J Syst Evol Microbiol 57, 81-91.
Li H. (2015) BFC: correcting illumina sequencing errors. arXiv:1502.03744.
Lopez-Siles M, et al. 2012. Cultured Representatives of Two Major Phylogroups of Human Colonic Faecalibacterium prausnitzii Can Utilize Pectin, Uronic Acids, and Host-Derived Substrates for Growth. Applied and Environmental Microbiology 78(2):420-428.
Seemann T. Prokka: rapid prokaryotic genome annotation. Bioinformatics. 2014;15:2068-9
Walker BJ, et al. Pilon: an integrated tool for comprehensive microbial variant detection and genome assembly improvement. PLoS One. 2014;9:e112963. doi: 10.1371/journal.pone.0112963.
Weizhong Li & Adam Godzik. Cd-hit: a fast program for clustering and comparing large sets of protein or nucleotide sequences", Bioinformatics, 2006; 22:1658-9.
Magoč T, Salzberg SL. FLASH: fast length adjustment of short reads to improve genome assemblies. Bioinformatics. 2011;27:2957-63.
Wang Q, Garrity GM, Tiedje JM, Cole JR. Naive Bayesian classifier for rapid assignment of rRNA sequences into the new bacterial taxonomy. Appl Environ Microbiol. 2007 Aug;73(16):5261-7.
Ouwerkerk J. P., Aalvink S., Belzer C., de Vos W. M. (2016). Akkermansia glycaniphila sp. nov., an anaerobic mucin-degrading bacterium isolated from reticulated python faeces. Int. J. Syst. Evol. Microbiol. 66, 4614-4620. 10.1099/ijsem.0.001399
Clinical breakpoints (Bacterial v9.0 and Fungal v9.0). The European Committee on Antimicrobial Susceptibility Testing: http://www.eucast.org/clinical _breakpoints. Published January 1, 2019 (bacteria).
Guo X, et al. (2016). Different subtype strains of Akkermansia muciniphila abundantly colonize in southern China. J Appl Microbiol. 2016 Feb;120(2):452-9. doi: 10.1111/jam.13022.
Piletz JE, Aricioglu F, Cheng JT, Fairbanks CA, Gilad VH, Haenisch B, Halaris A, Hong S, Lee JE, Li J, Liu P, Molderings GJ, Rodrigues AL, Satriano J, Seong GJ, Wilcox G, Wu N, Gilad GM. Agmatine: clinical applications after 100 years in translation. Drug Discov Today. 2013 Sep;18(17-18):880-93. doi: 10.1016/j.drudis.2013.05.017. Epub 2013 Jun 13. PMID: 23769988
Taksande BG, Gawande DY, Chopde CT, Umekar MJ, Kotagale NR. Agmatine ameliorates adjuvant induced arthritis and inflammatory cachexia in rats. Biomed Pharmacother. 2017 Feb;86:271-278. doi: 10.1016/j.biopha.2016.12.039. Epub 2016 Dec 19. PMID: 28006753.
Freitas AE, Bettio LE, Neis VB, Santos DB, Ribeiro CM, Rosa PB, Farina M, Rodrigues AL. Agmatine abolishes restraint stress-induced depressive-like behavior and hippocampal antioxidant imbalance in mice. Prog Neuropsychopharmacol Biol Psychiatry. 2014 Apr 3;50:143-50. doi: 10.1016/j.pnpbp.2013.12.012. Epub 2013 Dec 24. PMID: 24370459.
Cauwels, A., Rogge, E., Vandendriessche, B. et al. Extracellular ATP drives systemic inflammation, tissue damage and mortality. Cell Death Dis 5, e1102 (2014).
Chambers ES, Preston T, Frost G, Morrison DJ. Role of Gut Microbiota-Generated Short-Chain Fatty Acids in Metabolic and Cardiovascular Health. Curr Nutr Rep. 2018;7(4):198-206. doi:10.1007/s13668-018-0248-8
Ríos-Covián D, Ruas-Madiedo P, Margolles A, Gueimonde M, de los Reyes-Gavilán CG and Salazar N (2016) Intestinal Short Chain Fatty Acids and their Link with Diet and Human Health. Front. Microbiol. 7:185. doi: 10.3389/fmicb.2016.00185

### SEQUENCES

TTATGAGAGTTTGATCCTGGCTCAGGATGAACGCTGGCGGCGTGCTTAACACATGCAAGTCG
AACGAAGCATTTGCAACAGATTTCTTCGGATGAAGTTGCTTATGACTGAGTGGCGGACGGG
TGAGTAACGCGTGGGTAACCTGCCTTGTACTGGGGGATAGCAGCTGGAAACGGCTGGTAAT
ACCGCATAAGCGCACAATGTTGCATGACATGGTGTGAAAAACTCCGGTGGTATAAGATGGA
CCCGCGTCTGATTAGCTAGTTGGTGAGATAACAGCCCACCAAGGCGACGATCAGTAGCCGA
CCTGAGAGGGTGACCGGCCACATTGGGACTGAGACACGGCCCAGACTCCTACGGGAGGCAG
CAGTGGGGAATATTGCACAATGGAGGAAACTCTGATGCAGCGACGCCGCGTGAGTGAAGAA

GTAATTCGTTATGTAAAGCTCTATCAGCAGGGAAGATAGTGACGGTACCTGACTAAGAAGCT
CCGGCTAAATACGTGCCAGCAGCCGCGGTAATACGTATGGAGCAAGCGTTATCCGGATTTAC
TGGGTGTAAAGGGAGTGTAGGTGGCCATGCAAGTCAGAAGTGAAAATCCGGGGCTCAACCC
CGGAACTGCTTTTGAAACTGTAAGGCTAGAGTGCAGGAGGGGTGAGTGGAATTCCTAGTGT
AGCGGTGAAATGCGTAGATATTAGGAGGAACACCAGTGGCGAAGGCGGCTCACTGGACTGT
AACTGACACTGAGGCTCGAAAGCGTGGGGAGCAAACAGGATTAGATACCCTGGTAGTCCAC
GCCGTAAACGATGAATACTAGGTGTCGGGGCCCATAAGGGCTTCGGTGCCGCAGCAAACGC
AATAAGTATTCCACCTGGGGAGTACGTTCGCAAGAATGAAACTCAAAGGAATTGACGGGGA
CCCGCACAAGCGGTGGAGCATGTGGTTTAATTCGAAGCAACGCGAAGAACCTTACCAAGTC
TTGACATCCCACTGACCGGACAGTAATGTGTCCTTTCCTTCGGGACAGTGGTGACAGGTGGT
GCATGGTTGTCGTCAGCTCGTGTCGTGAGATGTTGGGTTAAGTCCCGCAACGAGCGCAACCC
CTATCCTTAGTAGCCAGCAGTAAGATGGGCACTCTAGGGAGACTGCCAGGGATAACCTGGA
GGAAGGTGGGGATGACGTCAAATCATCATGCCCCTTATGACTTGGGCTACACACGTGCTACA
ATGGCGTAAACAAAGTGAAGCGAAGTCGTGAGGCCAAGCAAATCACAAAAATAACGTCTCA
GTTCGGATTGTAGTCTGCAACTCGACTACATGAAGCTGGAATCGCTAGTAATCGCAGATCAG
AATGCTGCGGTGAATACGTTCCCGGGTCTTGTACACACCGCCCGTCACACCATGGGAGTCGA
AAATGCCCGAAGTCGGTGACCTAACGTAAGAAGGAGCCGCCGAAGGCAGGTTTGATAACTG
GGGTGAAGTCGTAACAAGGTAGCCGTATCGGAAGGTGCGGCTGGATCACCTCCTTT (SEQ ID
NO:1)

TATTGAGAGTTTGATCCTGGCTCAGGATGAACGCTGGCGGCGTGCTTAACACATGCAAGTCG
AACGGAACGCCAAGGAAAGAGTTTCGGACAATGGAATTGGCTGTTAGTGGCGGACGGGT
GAGTAACGCGTGAGTAACCTGCCTTGGAGTGGGGAATAACACAGTGAAAACTGTGCTAATA
CCGCATGACATATTGGTGTCGCATGGCGCTGATATCAAAGATTTATCGCTCTGAGATGGACT
CGCGTCTGATTAGATAGTTGGCGGGGTAACGGCCCACCAAGTCGACGATCAGTAGCCGGAC
TGAGAGGTTGGCCGGCCACATTGGGACTGAGACACGGCCCAGACTCCTACGGGAGGCAGCA
GTGGGGAATATTGGGCAATGGGCGCAAGCCTGACCCAGCAACGCCGCGTGAAGGAAGAAG
GCTTTCGGGTTGTAAACTTCTTTTAACAGGGACGAAGTAAGTGACGGTACCTGTTGAATAAG
CCACGGCTAACTACGTGCCAGCAGCCGCGGTAATACGTAGGTGGCAAGCGTTATCCGGATTT
ACTGGGTGTAAAGGGCGTGTAGGCGGGACTGCAAGTCAGATGTGAAAACTATGGGCTCAAC
CCATAGCCTGCATTTGAAACTGTAGTTCTTGAGTGTCGGAGAGGCAATCGGAATTCCGTGTG
TAGCGGTGAAATGCGTAGATATACGGAGGAACACCAGTGGCGAAGGCGGATTGCTGGACGA
TAACTGACGCTGAGGCGCGAAAGCGTGGGGAGCAAACAGGATTAGATACCCTGGTAGTCCA
CGCCGTAAACGATGGATACTAGGTGTGGGGGGTCTGACCCCCTCCGTGCCGCAGCTAACGCA
ATAAGTATCCCACCTGGGGAGTACGATCGCAAGGTTGAAACTCAAAGGAATTGACGGGGGC
CCGCACAAGCGGTGGAGTATGTGGTTTAATTCGAAGCAACGCGAAGAACCTTACCAGGGCT
TGACATCCTACTAACGAACCAGAGATGGATTAGGTGCCCTTCGGGGAAAGTAGAGACAGGT
GGTGCATGGTTGTCGTCAGCTCGTGTCGTGAGATGTTGGGTTAAGTCCCGCAACGAGCGCAA
CCCTTATTGTTAGTTGCTACGCAAGAGCACTCTAGCGAGACTGCCGTTGACAAAACGGAGGA
AGGTGGGGACGACGTCAAATCATCATGCCCCTTATGTCCTGGGCCACACACGTACTACAATG
GCGGTTAACAGAGGGAGGCAAAGCCGCGAGGCAGAGCAAACCCCTAAAAGCCGTCCCAGTT
CGGATTGCAGGCTGAAACCCGCCTGTATGAAGTCGGAATCGCTAGTAATCGCGGATCAGCAT
GCCGCGGTGAATACGTTCCCGGGCCTTGTACACACCGCCCGTCACACCATGAGAGTCGGGAA
CACCCGAAGTCCGTAGCCTAACTGCAAAGGGGGCGCGGCCGAAGGTGGGTTCGATAATTGG
GGTGAAGTCGTAACAAGGTAGCCGTATCGGAAGGTGCGGCTGGATCACCTCCTTT (SEQ ID
NO:2)

GAGATTAAAGATTTATCGGTGATGGATGGGGATGCGTCTGATTAGCTTGTTGGCGGGGTAAC
GGCCCACCAAGGCAACGATCAGTAGGGGTTCTGAGAGGAAGGTCCCCCACATTGGAACTGA
GACACGGTCCAAACTCCTACGGGAGGCAGCAGTGAGGAATATTGGTCAATGGACGAGAGTC
TGAACCAGCCAAGTAGCGTGCAGGATGACGGCCCTATGGGTTGTAAACTGCTTTTATAAGGG

AATAAAGTGAGTCTCGTGAGACTTTTGCATGTACCTTATGAATAAGGACCGGCTAATTCCG
TGCCAGCAGCCGCGGTAATACGGAAGGTCCGGGCGTTATCCGGATTTATTGGGTTTAAAGGG
AGCGTAGGCCGGAGATTAAGCGTGTTGTGAAATGTAGACGCTCAACGTCTGCACTGCAGCG
CGAACTGGTTTCCTTGAGTACGCACAAAGTGGGCGGAATTCGTGGTGTAGCGGTGAAATGCT
TAGATATCACGAAGAACTCCGATTGCGAAGGCAGCTCACTGGAGCGCAACTGACGCTGAAG
CTCGAAAGTGCGGGTATCGAACAGGATTAGATACCCTGGTAGTCCGCACGGTAAACGATGG
ATGCCCGCTGTTGGTCTGAACAGGTCAGCGGCCAAGCGAAAGCATTAAGCATCCACCTGG
GGAGTACGCCGGCAACGGTGAAACTCAAAGGAATTGACGGGGGCCCGCACAAGCGGAGGA
ACATGTGGTTTAATTCGATGATACGCGAGGAACCTTACCCGGGCTTGAATTGCAGAGGAAGG
ATTTGGAGACAATGACGCCCTTCGGGGTCTCTGTGAAGGTGCTGCATGGTTGTCGTCAGCTC
GTGCCGTGAGGTGTCGGCTTAAGTGCCATAACGAGCGCAACCCCTCTCCTTAGTTGCCATCA
GGTCAGGCTGGGCACTCTGGGGACACTGCCACCGTAAGGTGTGAGGAAGGTGGGGATGACG
TCAAATCAGCACGGCCCTTACGTCCGGGGCTACACACGTGTTACAATGGCAGGTACAGAGA
GACGGTTGTACGTAAGTACGATCAAATCCTTAAAGCCTGTCTCAGTTCGGACTGGGGTCTGC
AACCCGACCCCACGAAGCTGGATTCGCTAGTAATCGCGCATCAGCCATGGCGCGGTGAATA
CGTTCCCGGGCCTTGTACACACCGCCCGTCAAGCCATGAAAGCCGGGGGCGCCTAAAGTCCG
TGACCGTAAGGAGCGGCCTAGGGCGAAACTGGTAATTGGGGCTAAGTCGTAACAAGGTAGC
CGTACCGGAAGGTGCGGCTGGAACACCTCCTTT (SEQ ID NO:3)

## Claims

1. A composition comprising a biologically pure strain of an *Akkermansia sp.* deposited at the German Collection of Microorganisms and Cell Culture (DSM) under number DSM 33459.

2. The composition of claim 1, further comprising a biologically pure strain of *Intestinimonas massiliensis*; wherein the biologically pure strain of *I. massiliensis* is the *I. massiliensis* strain deposited at DSM under number DSM 33460.

3. A composition comprising isolated bacterial extracellular vesicles (EVs) derived from a biologically pure strain of an *Akkermansia sp.* deposited at DSM under number DSM 33459; optionally wherein the composition further comprises EVs derived from a biologically pure strain of *Intestinimonas massiliensis,* wherein the EVs derived from a biologically pure strain of *Intestinimonas massiliensis* are EVs derived from the *I. massiliensis* strain deposited at DSM under number DSM 33460.

4. The composition of any one of claims 1-3, wherein the composition is formulated for oral administration.

5. The composition of any one of claims 1-4, wherein the composition is lyophilized or freeze dried and/or wherein the composition is encapsulated or coated.

6. The composition of any one of claims 1-5, wherein the composition is a food product, food ingredient, dietary supplement, or medicament.

7. The composition of any one of claims 1-2 or 4-6, wherein at least about 1 x 10⁴ CFU/g composition to at least about 1 x 10¹² CFU/g composition of bacteria is present in the composition.

8. The composition of any one of claims 1-2 or 4-7, wherein the composition is a probiotic.

9. The composition of any one of claims 1-8, wherein the composition has been pasteurized or heat treated.

10. The composition of any one of claims 1-9, wherein the composition is a pharmaceutical composition and further comprises at least one pharmaceutically acceptable carrier and/or excipient.

11. A tablet, prolonged-release capsule, prolonged-release granule, powder, sachet, or gummy comprising the composition of any one of claims 1-10.

12. A kit comprising (a)(i) the composition of any one of claims 1-10; or (ii) the tablet, prolonged-release capsule, prolonged-release granule, powder, sachet, or gummy of claim 11 and b) written instructions for administration to a subject.

13. A method for making a composition comprising:
combining a biologically pure strain of *Intestinimonas massiliensis* and a biologically pure strain of an *Akkermansia sp.* deposited at DSM under number DSM 33459; or
deriving and combining extracellular vesicles (EVs) from a biologically pure strain of *Intestinimonas massiliensis* and a biologically pure strain of an *Akkermansia sp.* deposited at DSM under number DSM 33459;
wherein the *I. massiliensis* comprises the *I. massiliensis* strain deposited at DSM under number DSM 33460.

14. The method of claim 13, further comprising freeze frying or lyophilizing the composition.

15. A composition according to claims 1-11 for use in the prevention and/or treatment of one or more obesity-related disorders in a subject in need thereof, comprising administering the composition of any one of claims 1-10 or the tablet, prolonged-release capsule, prolonged-release granule, powder, sachet, or gummy of claim 11 to the subject; wherein the obesity related disorder is one or more disorders selected from the group consisting of obesity, metabolic syndrome, diabetes mellitus, insulin deficiency-related disorders, insulin-resistance related disorders, glucose intolerance, abnormal lipid metabolism, non-alcoholic fatty liver disease, hepatic steatosis, leptin resistance, reduced resistin levels, and/or cardiovascular disease.

## Patentansprüche

1. Zusammensetzung, umfassend einen biologisch reinen Stamm einer *Akkermansia sp.,* hinterlegt bei der DSMZ (Deutsche Sammlung von Mikroorganismen und Zellkulturen) unter der Nummer DSM 33459.

2. Zusammensetzung nach Anspruch 1, ferner umfassend einen biologisch reinen Stamm von *Intestinimonas massiliensis;* wobei es sich bei dem biologisch reinen Stamm von *I. massiliensis* um den bei der DSMZ unter der Nummer DSM 33460 hinterlegten *I. massiliensis*-Stamm handelt.

3. Zusammensetzung, umfassend isolierte bakterielle extrazelluläre Vesikel (EV), die von einem bei der DSMZ unter der Nummer DSM 33459 hinterlegten biologisch reinen Stamm einer *Akkermansia sp.* stammen; gegebenenfalls wobei die Zusammensetzung ferner EV umfasst, die von einem biologisch reinen Stamm von *Intestinimonas massiliensis* stammen, wobei es sich bei den von einem biologisch reinen Stamm von *Intestinimonas massiliensis* stammenden EV um EV handelt, die von dem bei der DSMZ unter der Nummer DSM 33460 hinterlegten *I. massiliensis*-Stamm stammen.

4. Zusammensetzung nach einem der Ansprüche 1-3, wobei die Zusammensetzung für die orale Verabreichung formuliert ist.

5. Zusammensetzung nach einem der Ansprüche 1-4, wobei die Zusammensetzung lyophilisiert oder gefriergetrocknet ist und/oder wobei die Zusammensetzung verkapselt oder beschichtet ist.

6. Zusammensetzung nach einem der Ansprüche 1-5, wobei es sich bei der Zusammensetzung um ein Lebensmittelprodukt, Lebensmittelzutat, Nahrungsergänzungsmittel oder Arzneimittel handelt.

7. Zusammensetzung nach einem der Ansprüche 1-2 oder 4-6, wobei wenigstens etwa 1 x 10⁴ CFU bis wenigstens etwa 1 x 10¹² CFU Bakterien pro Gramm Zusammensetzung in der Zusammensetzung vorliegen.

8. Zusammensetzung nach einem der Ansprüche 1-2 oder 4-7, wobei es sich bei der Zusammensetzung um ein Probiotikum handelt.

9. Zusammensetzung nach einem der Ansprüche 1-8, wobei die Zusammensetzung pasteurisiert oder wärmebehandelt wurde.

10. Zusammensetzung nach einem der Ansprüche 1-9, wobei es sich bei der Zusammensetzung um eine pharmazeutische Zusammensetzung handelt, die ferner wenigstens einen pharmazeutisch unbedenklichen Träger und/oder Exzipienten umfasst.

11. Tablette, Retardkapsel, Retardgranulat, Pulver, Sachet oder Gummi, umfassend die Zusammensetzung nach einem der Ansprüche 1-10.

12. Kit, umfassend (a) (i) die Zusammensetzung nach einem der Ansprüche 1-10 oder (ii) die Tablette, die Retardkapsel, das Retardgranulat, das Pulver, das Sachet bzw. das Gummi nach Anspruch 11 und b) schriftliche Anweisungen für eine Verabreichung an ein Individuum.

13. Verfahren zur Herstellung einer Zusammensetzung, umfassend:
Kombinieren eines biologisch reinen Stamms von *Intestinimonas massiliensis* und eines bei der DSMZ unter der Nummer DSM 33459 hinterlegten biologisch reinen Stamms einer *Akkermansia sp.;* oder
Gewinnen und Kombinieren extrazellulärer Vesikel (EV) von einem biologisch reinen Stamm von *Intestinimonas massiliensis* und einem bei der DSMZ unter der Nummer DSM 33459 hinterlegten biologisch reinen Stamm einer *Akkermansia sp.;*
wobei das *I. massiliensis* den bei der DSMZ unter der Nummer DSM 33460 hinterlegten *I. massiliensis-*Stamm umfasst.

14. Verfahren nach Anspruch 13, ferner umfassend Gefriertrocknen oder Lyophilisieren der Zusammensetzung.

15. Zusammensetzung gemäß Anspruch 1-11 zur Verwendung bei der Vorbeugung und/oder Behandlung einer oder mehrerer Störungen in Verbindung mit Fettleibigkeit bei einem behandlungsbedürftigen Individuum, umfassend Verabreichen der Zusammensetzung nach einem der Ansprüche 1-10 oder der Tablette, der Retardkapsel, des Retardgranulats, des Pulvers, des Sachet bzw. des Gummis nach Anspruch 11 an das Individuum; wobei es sich bei der Störung in Verbindung mit Fettleibigkeit um eine oder mehrere Störungen handelt, die aus der Gruppe bestehend aus Fettleibigkeit, metabolischem Syndrom, Diabetes mellitus, Störungen in Verbindung mit Insulinmangel, Störungen in Verbindung mit Insulinresistenz, Glukoseintoleranz, anomalem Fettstoffwechsel, nichtalkoholischer Fettlebererkrankung, Steatosis hepatis, Leptinresistenz, verringerten Resistinspiegeln und/oder Herz-Kreislauf-Krankheit ausgewählt sind.

## Revendications

1. Composition comprenant une souche biologiquement pure d'un Akkermansia sp. déposée à la collection allemande de micro-organismes et de culture cellulaire (DSM) sous le numéro DSM 33459.

2. Composition selon la revendication 1, comprenant en outre une souche biologiquement pure d'Intestinimonas massiliensis ; la souche biologiquement pure de I. massiliensis étant la souche de I. massiliensis déposée à la DSM sous le numéro DSM 33460.

3. Composition comprenant des vésicules extracellulaires bactériennes isolées (VE) issues d'une souche biologiquement pure d'un Akkermansia sp. déposée à la DSM sous le numéro DSM 33459 ; éventuellement la composition comprenant en outre des VE issues d'une souche biologiquement pure d'Intestinimonas massiliensis, les VE issues d'une souche biologiquement pure d'Intestinimonas massiliensis étant des EV issues de la souche de I. massiliensis déposée à la DSM sous le numéro DSM 33460.

4. Composition selon l'une quelconque revendication 1 à 3, dans laquelle la composition est formulée pour une administration orale.

5. Composition selon l'une quelconque revendication 1 à 4, dans laquelle la composition est lyophilisée ou séchée à froid et/ou la composition est encapsulée ou enrobée.

6. Composition selon l'une quelconque des revendications 1 à 5, dans laquelle la composition est un produit alimentaire, un ingrédient alimentaire, un supplément diététique ou un médicament.

7. Composition selon l'une quelconque revendication 1 à 2 ou 4 à 6, dans laquelle au moins environ 1 x 10⁴ UFC/g de composition à au moins environ 1 x 10¹² UFC/g de composition de bactéries est présente dans la composition.

8. Composition selon l'une quelconque revendication 1 à 2 ou 4 à 7, dans laquelle la composition est un probiotique.

9. Composition selon l'une quelconque des revendications 1 à 8, dans laquelle la composition a été pasteurisée ou traitée thermiquement.

10. Composition selon l'une quelconque des revendications 1 à 9, dans laquelle la composition est une composition pharmaceutique et comprend en outre au moins un support et/ou excipient pharmaceutiquement acceptable.

11. Comprimé, capsule à libération prolongée, granule à libération prolongée, poudre, sachet ou gomme comprenant la composition selon l'une quelconque des revendications 1 à 10.

12. Kit comprenant (a)(i) la composition selon l'une quelconque des revendications 1-10 ; ou (ii) le comprimé, la capsule à libération prolongée, le granule à libération prolongée, la poudre, le sachet ou la gomme selon la revendication 11 et b) des instructions écrites pour une administration à un sujet.

13. Procédé pour la préparation d'une composition comprenant :
la combinaison d'une souche biologiquement pure d'Intestinimonas massiliensis et d'une souche biologiquement pure d'un Akkermansia sp. déposée à la DSM sous le numéro DSM 33459 ; ou
la dérivation et la combinaison de vésicules extracellulaires (VE) d'une souche biologiquement pure d'Intestinimonas massiliensis et d'une souche biologiquement pure d'un Akkermansia sp. déposée à la DSM sous le numéro DSM 33459 ;
l'I. massiliensis comprenant la souche d'I. massiliensis déposée à la DSM sous le numéro DSM 33460.

14. Procédé selon la revendication 13, comprenant en outre le séchage par congélation ou la lyophilisation de la composition.

15. Composition selon les revendications 1 à 11 pour une utilisation dans la prévention et/ou le traitement d'un ou de plusieurs troubles liés à l'obésité chez un sujet qui en a besoin, comprenant une administration de la composition selon l'une quelconque des revendications 1 à 10 ou du comprimé, de la capsule à libération prolongée, du granule à libération prolongée, de la poudre, du sachet ou de la gomme selon la revendication 11 au sujet ; le trouble lié à l'obésité étant un ou plusieurs troubles choisis dans le groupe constitué par l'obésité, le syndrome métabolique, le diabète sucré, les troubles liés à une carence en insuline, les troubles liés à la résistance à l'insuline, l'intolérance au glucose, le métabolisme lipidique anormal, la maladie du foie gras non alcoolique, la stéatose hépatique, la résistance à la leptine, la réduction des niveaux de résistine et/ou une maladie cardiovasculaire.
